# EUROPEAN PATENT APPLICATION

(11) **EP 3 747 957 A1**
(43) Date of publication of application: **09.12.2020**
(21) Application number: 19179207.6
(22) Date of filing: 07.06.2019
(51) Int. Cl.: C09B 57/02, C07D 409/04, C07D 409/14, G01N 33/533, C12Q 1/37

(54) **FLUORESCENT VINYL TIOPHENE AND BITIOPHENE COUMARINS DYES AND METHOD OF SYNTHESIS THEREOF**

(71) Applicant: Universidade de Évora, 7000-803 Évora (PT)
(72) Inventor: PEREIRA, Antonio, 7000-890 Évora (PT); MARTINS, Sérgio, 7000-513 Évora (PT); CALDEIRA, Ana, 7000-651 Évora (PT); CANDEIAS, António, 7000-651 Évora (PT); EUSTÁQUIO, Raquel, 7090-027 Alcáçovas (PT); SILVA, Sofia, 7090-414 Aguiar (PT)
(74) Representative: Gata-Goncalves, Ligia

(57) **Abstract**

The present invention relates to fluorescent vinyl thiophene and bithiophene coumarins, in particular to 7-alkylamino-3-vinyl thiophene and 7-alkylamino-3-vinyl dithiophene coumarin compounds of general formula (I), their derivatives, method of synthesis and uses thereof as fluorescent dyes for tagging biomolecules and as dye-sensitized solar cells (DSSCs).

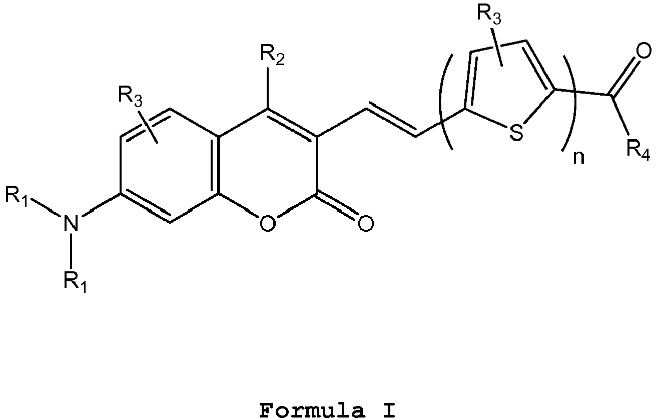

Therefore, the present invention is in the technical domain of biochemistry, molecular biology and biology and is useful to be applied in research and diagnosis, including medical diagnosis.

## Description

### FIELD OF THE INVENTION

The present invention relates to fluorescent vinyl tiophene and bitiophene coumarins, in particular to 7-alkylamino-3-vinyl tiophene and 7-alkylamino-3-vinyl ditiophene coumarin compounds of general formula (I), their derivatives, method of synthesis and uses thereof as fluorescent dyes for tagging biomolecules and as dye-sensitized solar cells (DSSCs).

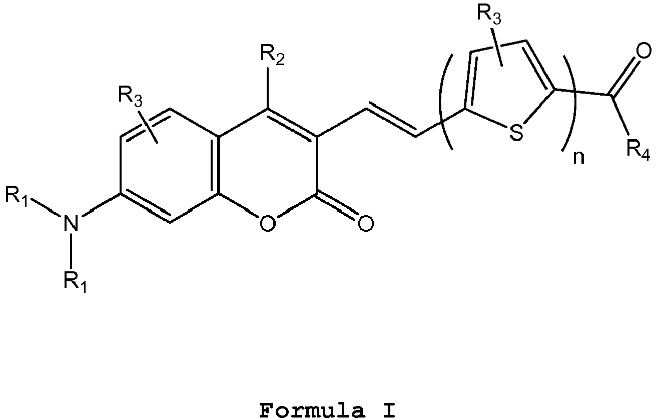

Therefore, the present invention is in the technical domain of biochemistry, molecular biology and biology and is useful to be applied in research and diagnosis, including medical diagnosis. In photochemistry, the DSSC compounds are useful for generating electrical energy.

### BACKGROUND OF THE INVENTION

Coumarins have been extensively described in the literature. Coumarin derivatives represent one of the most important chemical classes of organic fluorescent materials being one of the most extensively investigated and commercially significant groups of organic fluorescent materials.

In the last two decades a great amount of publications on the development of coumarin derivatives as photosensitizers for DSSCs (Dye-Sensitized Solar Cells) and as fluorescent labels for biomolecules has been reported.

Some of organic dyes actually used as photosensitizers for DSSCs and as fluorescent labels, involve complex reactions with low yields which makes this kind of compounds very expensive.

The development of effective sensitizers for DSSCs and fluorescent labels for biomolecules involves a system Donor-π bridge-Acceptor molecules.

Coumarins with electron-donating substituents introduced in position 7 and electron-withdrawing moieties in position 3, allows a further decrease in the energy gap between the highest occupied molecular orbital (HOMO) and the lowest unoccupied molecular orbital (LUMO).

This extends dye absorption bands to longer wavelengths and increases their molar extinction coefficients.

EP3219712 A1 entitled "COUMARIN DYES AND CONJUGATES THEREOF" describes the synthesis of 7-alkylamino-3-(thienyl) coumarin fluorescent dyes. The synthetic strategy of these dyes does not involve a system Donor-π bridge-Acceptor. The delocalization of π electrons between the coumarin moiety and the thiophene group, directly bonded in position 3, is very limited due the problematic orbital coalescence between these two groups. The use of these compounds as chromophores presents many limitations due to the low wavelengths produced.

Since the work of Hara *et al.* [K. Hara, M. Kurashige, Y. Danoh, C. Kasada, A. Shinpo, S. Suga, K. Sayama, H. Arakawa, Design of new coumarin dyes having thiophene moieties for highly efficient organic-dye-sensitized solar cells, New J. Chem. 27 (5) (2003) 783-785], where coumarins having thiophene moieties were developed for DSSCs with a conversion efficiency (h) of 7.7%, several authors devoted their attention to the coumarin scaffold.

US4956480 entitled "7-amino-4-methyl-coumarin-3-carboxyalkyl derivates and fluorescent conjugates thereof" describes coumarin compounds for use as fluorescent labelling agents. The related synthesis of these compounds is afforded by different synthetic strategy at 3-position of the coumarin.

US8431416 describes halogenated 7-hydroxy coumarin dyes that can be used as fluorescent labels.

Fluorescence of 7-hydroxy coumarins is pH dependent due their high pKa value of the C7-hydroxyl proton.

The protonated or neutral form of these dyes is not fluorescent. For these dyes to become fluorescent, complete deprotonation of the 7-hydroxyl proton is required to give the deprotonated or anionic form. At physiological pH these dyes exist in their neutral form and are not fluorescent and therefore not useful in biological assays.

7-amino coumarins are known to be pH independent over a much wider pH range and show excellent photostability and high fluorescence quantum yields. The primary amine derivatives, such as described in US5696157 show absorption maxima near the UV (350nm).

Some of 7-amino coumarins derivatives are water insoluble and extremely hydrophobic, producing high levels of quenching when conjugated to an analyte.

In view of the above, there is still the need to provide new compounds, as photosensitizers for dye-sensitized solar cells and as fluorescent labels for biomolecules and methods for obtaining said compounds with high yields, easy to isolate and low coast.

The present invention proposes to overcome the problems of the prior art by providing novel tiophene and bitiophene coumarin compounds obtained by a simple, effective and high reaction yield synthesis, which is independent of the pH, requiring low production costs, the resulting compounds presenting good photochemical and thermal stability and maximum absorption at high wavelengths.

### SUMMARY OF THE INVENTION

The present invention relates to fluorescent vinyl tiophene and bitiophene coumarins of formulae (I), their derivatives, and method of synthesis thereof. Further, the present invention also relates to the use of said compounds as dye-sensitized solar cells and to biosensors comprising said compounds.

In a first aspect, the present invention relates to fluorescent 7-alkylamino-3-vinyl tiophene and 7-alkylamino-3-vinyl ditiophene coumarin compounds of formulae I **according to claim 1**.

These compounds present good photochemical and thermal stability and maximum absorption at high wavelengths making them suitable to be used **as fluorescent dyes** for tagging biomolecules, thus allowing identifying and quantifying such molecules or **as dye-sensitized solar cells** (DSSCs) for energy production.

The fluorescent vinyl tiophene and bitiophene coumarins of formulae (I), their derivatives and intermediary compounds are obtained by the methods disclosed herein **according to claim 7 or 8.**

These methods revealed to be simple, effective and result in high reaction yield synthesis, which is independent of the pH, requiring low production costs.

A further aspect of the invention relates to the uses of fluorescent 7-alkylamino-3-vinyl tiophene and 7-alkylamino-3-vinyl ditiophene coumarin compounds of formulae I **as fluorescent dyes for tagging biomolecules,** thus allowing identifying and quantifying such molecules or **as dye-sensitized solar cells** (DSSCs) for energy production.

### DESCRIPTION OF THE FIGURES

The following figures show UV spectra of some derivatives of 7-alkylamino-3-vinyl tiophene and 7-alkylamino-3-vinyl ditiophene coumarin compounds of general formula (I), according to the present invention:

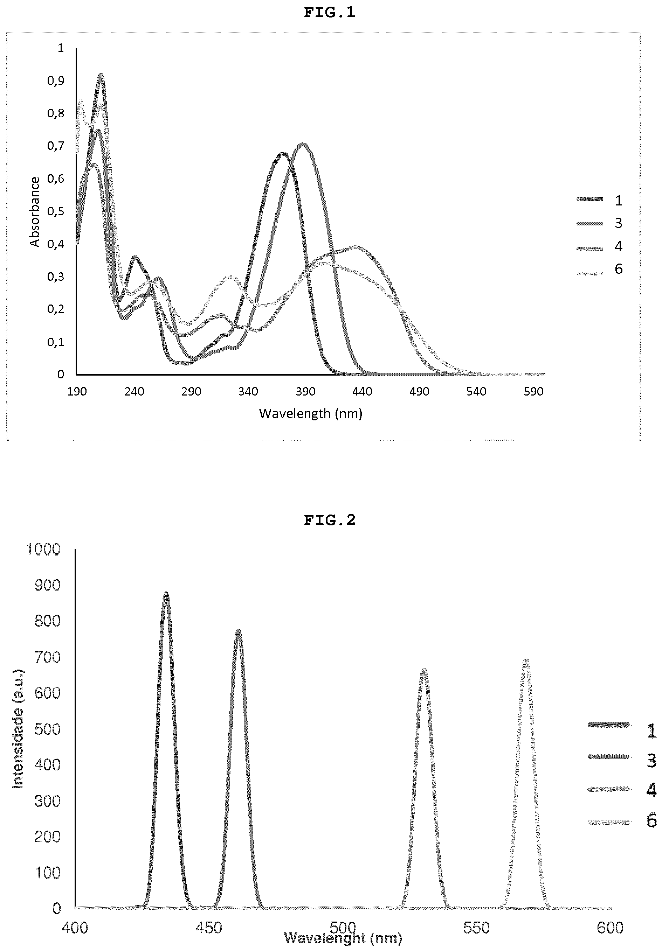

**Figure 1****.** Show the UV spectra (Abs) of the following compounds:
   7-diethylamino-4-methylcoumarin (**1**),
   7-diethylamino-4-methyl-3-vinylcoumarin (**3**),
   (*E*)-methyl 5-(2-(7-(diethylamino)-4-methyl-coumarin-3-yl)vinyl)-thiophene-2-carboxylate (**4**), and
   sodium (*E*/*Z*)-4-((5-(2-(7-(diethylamino)-4-methyl-coumarin-3-yl)vinyl)thiophene-2-carbonyl)oxy)-2,3,5,6-tetrafluorobenzenesulfonate (**6**).
**Figure 2****.** Show the UV spectra (Em) of the following compounds:
   7-diethylamino-4-methylcoumarin (**1**),
   7-diethylamino-4-methyl-3-vinylcoumarin(**3**),
   (*E*)-methyl 5-(2-(7-(diethylamino)-4-methyl-coumarin-3-yl)vinyl)-thiophene-2-carboxylate (**4**), and
   sodium (*E*/*Z*)-4-((5-(2-(7-(diethylamino)-4-methyl-coumarin-3-yl)vinyl)thiophene-2-carbonyl)oxy)-2,3,5,6-tetrafluorobenzenesulfonate (**6**).

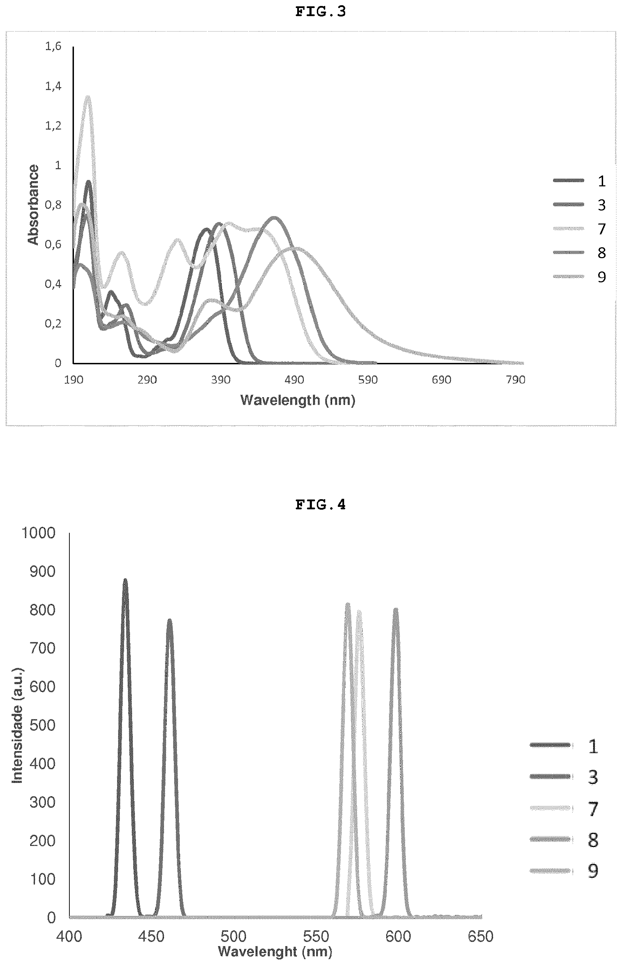

**Figure 3****.** Show the UV spectra (Abs) of the following compounds:
   7-diethylamino-4-methylcoumarin (**1**),
   7-diethylamino-4-methyl-3-vinylcoumarin (**3**),
   (*E*)-5-(2-(7-(diethylamino)-4-methyl-coumarin-3-yl)vinyl)thiophene-2-carbaldehyde (**7**),
   (*E*)-5'-(2-(7-(diethylamino)-4-methyl-coumarin-3-yl)vinyl)-[2,2'-bithiophene]-5-carbaldehyde (**8**), and
   (*E*)-2-cyano-3-(5-((*E*)-2-(7-(diethylamino)-4-methyl-coumarin-3-yl)vinyl)thiophen-2-yl)acrylic acid (**9**).
   **Figure 4****.** Show the UV spectra (Em) of the following compounds:
      7-diethylamino-4-methylcoumarin (**1**),
      7-diethylamino-4-methyl-3-vinylcoumarin (**3**),
      (*E*)-5-(2-(7-(diethylamino)-4-methyl-coumarin-3-yl)vinyl)thiophene-2-carbaldehyde (**7**),
      (*E*)-5'-(2-(7-(diethylamino)-4-methyl-coumarin-3-yl)vinyl)-[2,2'-bithiophene]-5-carbaldehyde (**8**), and
      (E)-2-cyano-3-(5-((*E*)-2-(7-(diethylamino)-4-methyl-coumarin-3-yl)vinyl)thiophen-2-yl)acrylic acid (**9**).

### GENERAL DESCRIPTION OF THE INVENTION

The present invention relates to fluorescent vinyl tiophene and bitiophene coumarins, in particular to 7-alkylamino-3-vinyl tiophene and 7-alkylamino-3-vinyl ditiophene coumarin compounds of general formula (I), their derivatives, method of synthesis and uses thereof as fluorescent dyes for tagging biomolecules and as dye-sensitized solar cells (DSSCs). 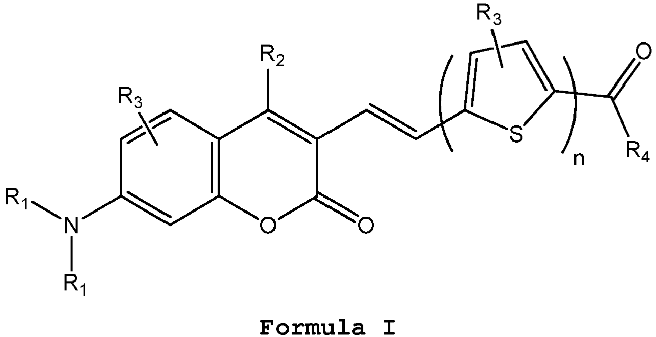 wherein:
n is 1 or 2,
R₁ and R₂ are independently -(CH₂)ₙ-CH₃, and n is 0, 1, 2 or 3,
R₃ is independently H or SO₃⁻ Na⁺,
R₄ is H or OCH₃,

Compounds of formulae (II), (III), (IV), (V) and (VI) below can be used as fluorescent dyes for tagging biomolecules: 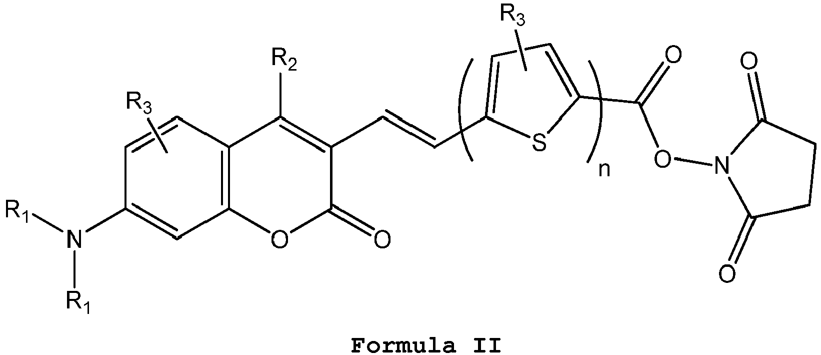 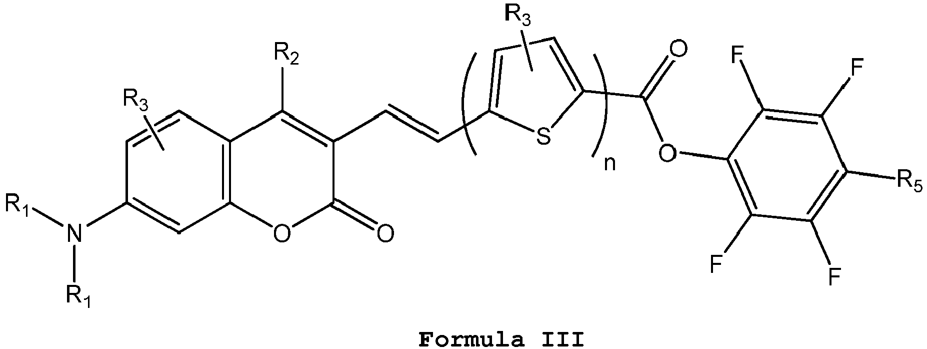 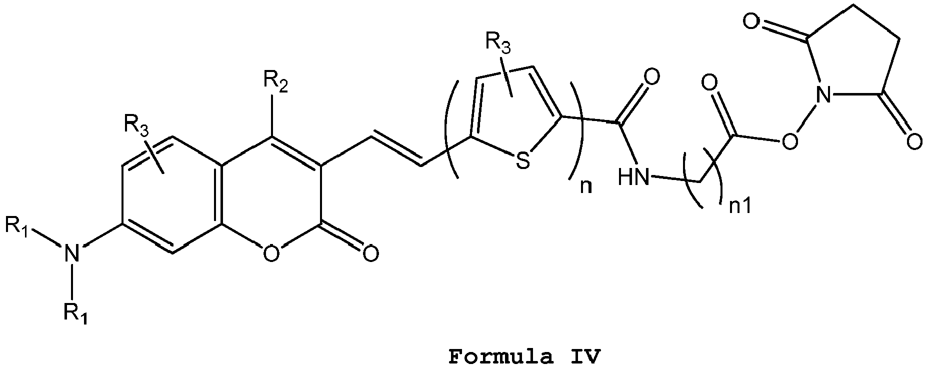 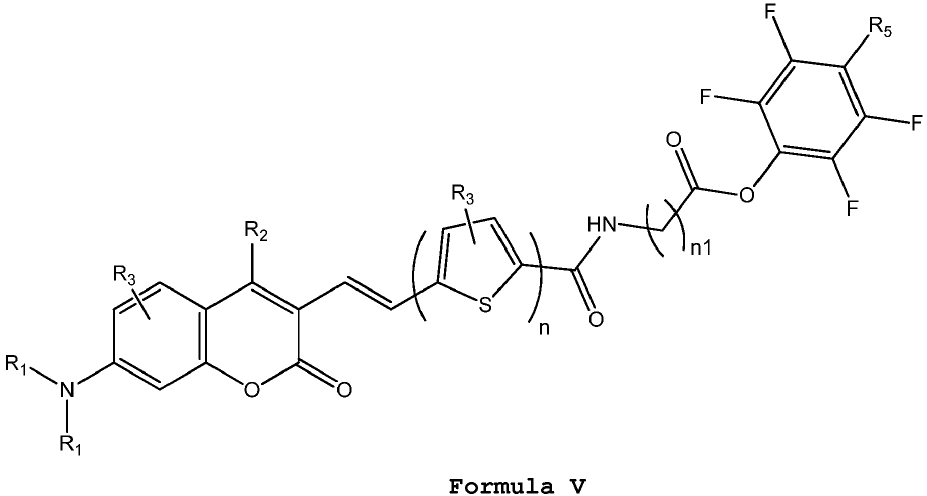 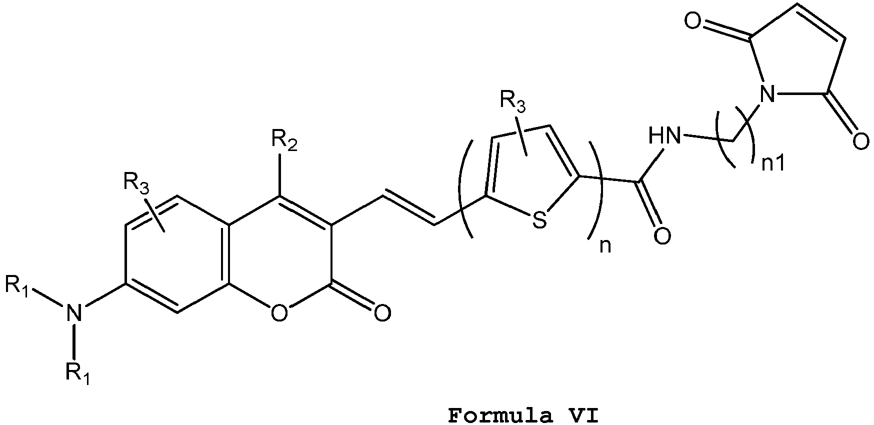 wherein:
- n: is 1 or 2,
- n₁: is 1, 2, 3, 4 or 5,
- R₁: is -(CH₂)ₙ-CH₃, and n is 0, 1, 2 or 3,
- R₂: is -(CH₂)ₙ-CH₃, and n is 0 or 1,
- R₃: is independently H or SO₃⁻ Na⁺,
- R₅: is H, F or SO₃⁻ Na⁺,

Compounds of formulae (VII) below can be used as dye-sensitized solar cells (DSSCs): 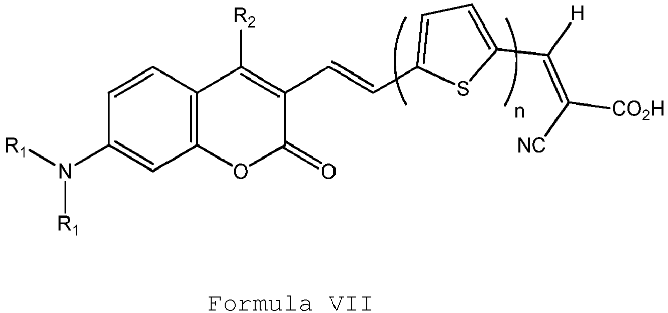 wherein:
- n: is 1 or 2,
- R₁: is -(CH₂)ₙ-CH₃, and n is 0, 1, 2 or 3,
- R₂: is -(CH₂)ₙ-CH₃, and n is 0 or 1,

In one specific embodiment, compounds of formulae (II), (III), (IV), (V) and (VI) are particularly useful to **be used as fluorescent labels for biomolecules.**

In another specific embodiment, compounds of formula (VII) are particularly useful to be used as DSSC.

### 1. Method for producing coumarin compounds derivatives of formulae (II), (III), (IV), (V) and (VI)

**Fluorescent labels for biomolecules** based on coumarin derivatives are obtained by a process comprising the following steps:
a) **reacting compounds of formula (VIII)** 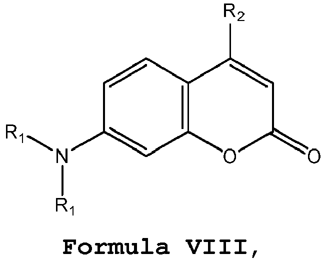 wherein:
   - R₁: is -(CH2)n-CH3, wherein n is 0, 1, 2 or 3; and
   - R₂: is -(CH2)n-CH3, wherein n is 0 or 1,
   with bromine in a reaction solvent selected from the group comprising chloroform, dichloromethane, 1,2-dichloroethane, acetonitrile, a mixed solvent thereof and the like, under stirring at a temperature between 0°C and 5°C for 10 hours to 24 hours;
b) adding to the **reactional mixture** a base selected from the group comprising triethylamine, 4-(dimethylamino)pyridine, piperidine, piperazine, pyridine, 1,4-diazabicyclo[2.2.2]octane (DABCO), 1,8-diazabicyclo[5.4.0]undec-7-ene (DBU) and the like, and stirring the reactional mixture at a temperature between 20°C and 25°C for 30 minutes to 3 hours; and
   evaporating the reaction mixture to dryness and purifying the residue **to produce compounds of formula (IX);** 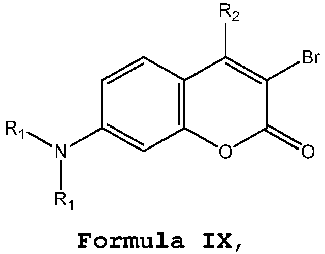 wherein:
   - R₁: is -(CH2)n-CH3, wherein n is 0, 1, 2 or 3; and
   - R₂: is -(CH₂)ₙ-CH₃, and n is 0 or 1.
c) **reacting compounds of formula (IX)** with potassium vinyltrifluoroborate and [1,1'-bis (diphenylphosphino)ferrocene]dichloropalladium(II) complex with dichloromethane in a reaction solvent selected from the group comprising n-propanol, 2-propanol, ethanol, tert-butanol, 2-butanol, acetonitrile, a mixed solvent thereof and the like, and stirring under inert atmosphere at a temperature between 78°C and 98°C for 15 minutes to 2 hours; and
   evaporating the reaction mixture to dryness and purifying the residue **to produce compounds of formula (X)** 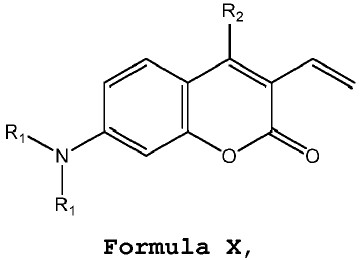 wherein:
   - R₁: is -(CH2)n-CH3, wherein n is 0, 1, 2 or 3; and
   - R₂: is -(CH₂)ₙ-CH₃, and n is 0 or 1.
d) **reacting compounds of formula (X)** with methyl 5-bromothiophene-2-carboxylate (or methyl 5'-bromo-[2,2'-bithiophene]-5-carboxylate),tetrakis(triphenylphosphine)palladium(0) and silver acetate in a reaction solvent selected from the group comprising N,N-dimethylformamide, dimethyl sulfoxide, a mixed solvent thereof and the like, and stirring under inert atmosphere at a temperature between 78°C and 98°C for 24 hours to 72 hours; and
   evaporating the reaction mixture to dryness and purifying the residue to **produce compounds of formula (XI)** 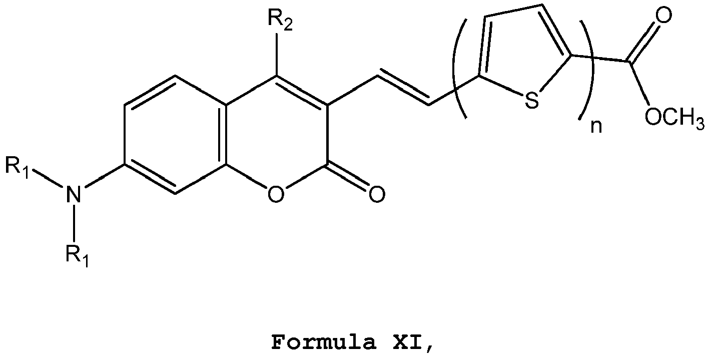 wherein:
   - n: is 1 or 2
   - R₁: is -(CH2)n-CH3, wherein n is 0, 1, 2 or 3; and
   - R₂: is -(CH₂)ₙ-CH₃, and n is 0 or 1.
e) **reacting compounds of formula (XI)** with 1 M sodium hydroxide in a reaction solvent selected from the group comprising 1,4-dioxane, N,N-dimethylformamide, dimethyl sulfoxide, a mixed solvent thereof and the like, and stirring at a temperature between 50°C and 70°C for 1 hour to 2 hours and neutralizing with HCl (10%) solution; and evaporating the reaction mixture to dryness and purifying the residue to **produce compounds of formula (XII)** 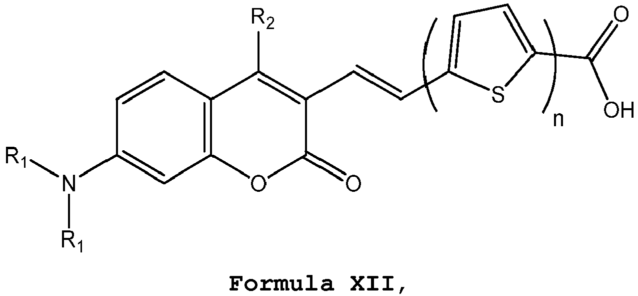 wherein
   - n: is 1 or 2
   - R₁: is -(CH2)n-CH3, wherein n is 0, 1, 2 or 3; and
   - R₂: is -(CH₂)ₙ-CH₃, and n is 0 or 1.

Compounds **of formula (XII)** of the present invention allow for producing new **fluorescent labels for biomolecules of general formulas (II), (III), (IV), (V) and (VI)** by a process comprising the following steps:
f) **reacting compounds of formula (XII)** with N-hydroxysuccinimide, and N,N'-dicyclohexylcarbodiimide in a reaction solvent selected from the group comprising acetonitrile and *N,N*-dimethylformamide, or dimethyl sulfoxide, a mixed solvent thereof and the like, and stirring at a temperature between 20°C and 26°C for 24 hours to 72 hours; and
   evaporating the reaction mixture to dryness and purifying the residue to **produce compounds of formula (XIII).** 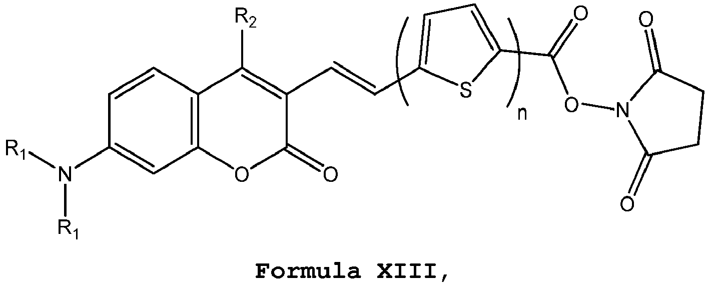 wherein:
   - n: is 1 or 2,
   - R₁: is -(CH2)n-CH3, wherein n is 0, 1, 2 or 3; and
   - R₂: is -(CH₂)ₙ-CH₃, and n is 0 or 1.
g) **reacting compounds of formula (XIII)** with fuming sulfuric acid (30% SO3), and stirring at a temperature between 20 °C and 26 °C for 30 minutes to 1 hour; and adding NaCl until no further precipitate formation; and recrystallizing the reaction mixture in acetonitrile to **produce compounds of formula (II).** 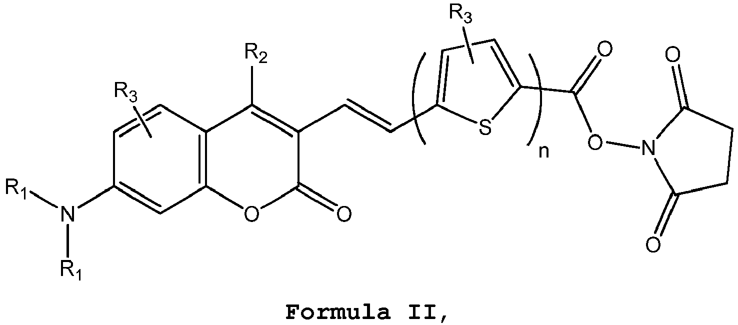 wherein:
   - n: is 1 or 2
   - R₁: is -(CH2)n-CH3, wherein n is 0, 1, 2 or 3;
   - R₂: is -(CH₂)ₙ-CH₃, and n is 0 or 1, and
   - R₃: is independently H or SO₃⁻ Na⁺.
h) **reacting compounds of formula (XII)** with 4-sulfotetrafluorophenol sodium salt (or pentafluorophenol, or 2,3,5,6-tetrafluorophenol), and N,N'-dicyclohexylcarbodiimide in a reaction solvent selected from the group comprising acetonitrile and N,N-dimethylformamide, or dimethyl sulfoxide, a mixed solvent thereof and the like, and stirring at a temperature between 20°C and 26°C for 24 hours to 72 hours; and evaporating the reaction mixture to dryness and purifying the residue to **produce compounds of formula (XIV).** 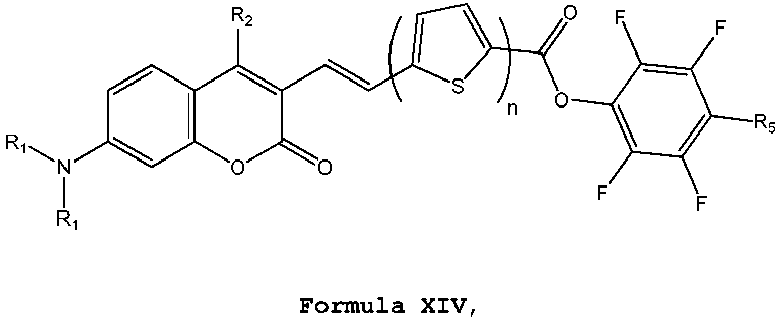 wherein:
   - n: is 1 or 2
   - R₁: is -(CH2)n-CH3, wherein n is 0, 1, 2 or 3;
   - R₂: is -(CH₂)ₙ-CH₃, and n is 0 or 1; and
   - R₅: is H, F or SO₃⁻ Na⁺.
i) **reacting compounds of formula (XIV)** with fuming sulfuric acid (30% SO3), and stirring at a temperature between 20 °C and 26 °C for 30 minutes to 1 hour; and adding NaCl until no further precipitate formation; and recrystallizing the reaction mixture in acetonitrile to **produce compounds of formula (III)**. 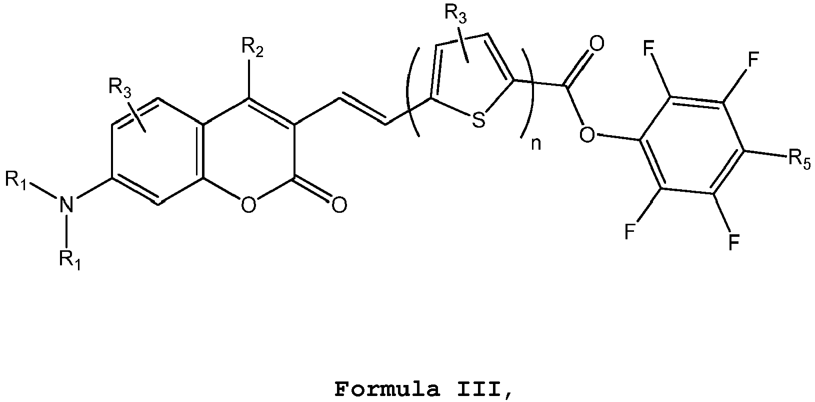 wherein:
   - n: is 1 or 2
   - R₁: is -(CH2)n-CH3, wherein n is 0, 1, 2 or 3;
   - R₂: is -(CH₂)ₙ-CH₃, and n is 0 or 1;
   - R₃: is independently H or SO₃⁻ Na⁺, and
   - R₅: is H, F or SO₃⁻ Na⁺.
j) **reacting compounds of formula (XII)** with methyl 2-aminoacetate (or methyl 3-aminopropanoate, or methyl 4-aminobutanoate, or methyl 5-aminopentanoate, or methyl 6-aminohexanoate), and N,N'-dicyclohexylcarbodiimide in a reaction solvent selected from the group comprising acetonitrile and N,N-dimethylformamide, or dimethyl sulfoxide, a mixed solvent thereof and the like, and stirring at a temperature between 20 °C and 26 °C for 24 hours to 72 hours; and
   evaporating the reaction mixture to dryness and purifying the residue to **produce compounds of formula (XV).** 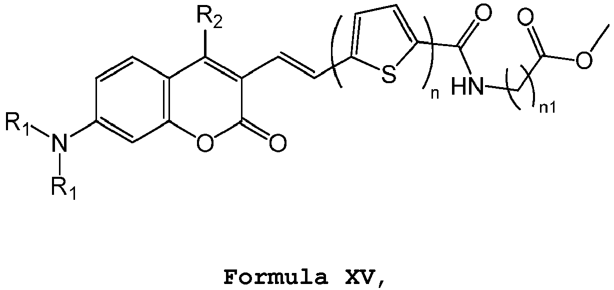 wherein:
   - n: is 1 or 2
   - n₁: is, 1, 2, 3, 4 or 5
   - R₁: is -(CH2)n-CH3, wherein n is 0, 1, 2 or 3; and
   - R₂: is -(CH₂)ₙ-CH₃, and n is 0 or 1.
l) **reacting a compounds of formula (XV)** with 1 M sodium hydroxide in a reaction solvent selected from the group comprising 1,4-dioxane, N,N-dimethylformamide, dimethyl sulfoxide, a mixed solvent thereof and the like, and stirring at a temperature between 50 °C and 70 °C for 1 hour to 2 hours and neutralizing with HCl (10%) solution; and
   the reaction mixture to dryness and purifying the residue to **produce compounds of formula (XVI).** 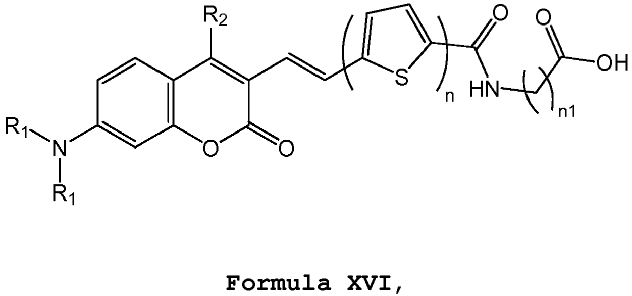 wherein:
   - n: is 1 or 2;
   - n₁: is, 1, 2, 3, 4 or 5;
   - R₁: is -(CH2)n-CH3, wherein n is 0, 1, 2 or 3; and
   - R₂: is -(CH₂)ₙ-CH₃, and n is 0 or 1.
m) **reacting compounds of formula (XVI)** with *N-*hydroxysuccinimide, and N,N'-dicyclohexylcarbodiimide in a reaction solvent selected from the group comprising acetonitrile and *N,N*-dimethylformamide, or dimethyl sulfoxide, a mixed solvent thereof and the like, and stirring at a temperature between 20 °C and 26 °C for 24 hours to 72 hours; and
   evaporating the reaction mixture to dryness and purifying the residue to **produce compounds of formula (XVII).** 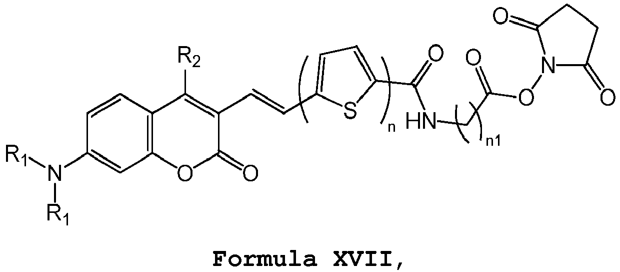 Wherein:
   - n: is 1 or 2
   - n₁: is, 1, 2, 3, 4 or 5
   - R₁: is -(CH2)n-CH3, wherein n is 0, 1, 2 or 3; and
   - R₂: is -(CH₂)ₙ-CH₃, and n is 0 or 1.
n) **reacting compounds of formula (XVII)** with fuming sulfuric acid (30% SO3), and stirring at a temperature between 20°C and 26 °C for 30 minutes to 1 hour; and adding NaCl until no further precipitate formation; and
   recrystallizing the reaction mixture in acetonitrile to **produce compounds of formula (IV).** 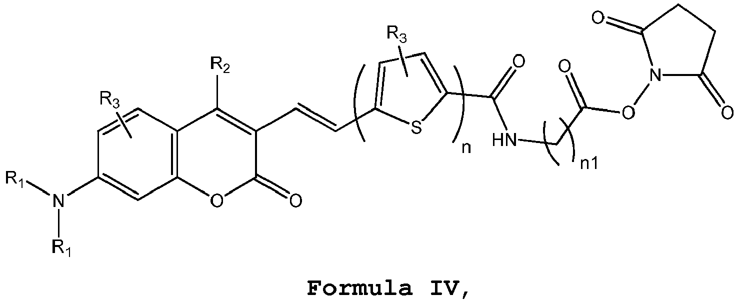 wherein:
   - n: is 1 or 2
   - n1: is, 1, 2, 3, 4 or 5
   - R₁: is -(CH2)n-CH3, wherein n is 0, 1, 2 or 3;
   - R₂: is -(CH₂)ₙ-CH₃, and n is 0 or 1; and
   - R₃: is independently H or SO₃⁻ Na⁺.
o) **reacting compounds of formula (XVI)** with 4-sulfotetrafluorophenol sodium salt (or pentafluorophenol, or 2,3,5,6-tetrafluorophenol), and N,N'-dicyclohexylcarbodiimide in a reaction solvent selected from the group comprising acetonitrile and N,N-dimethylformamide, or dimethyl sulfoxide, a mixed solvent thereof and the like, and stirring at a temperature between 20 °C and 26 °C for 24 hours to 72 hours; and evaporating the reaction mixture to dryness and purifying the residue to **produce compounds of formula (V).** 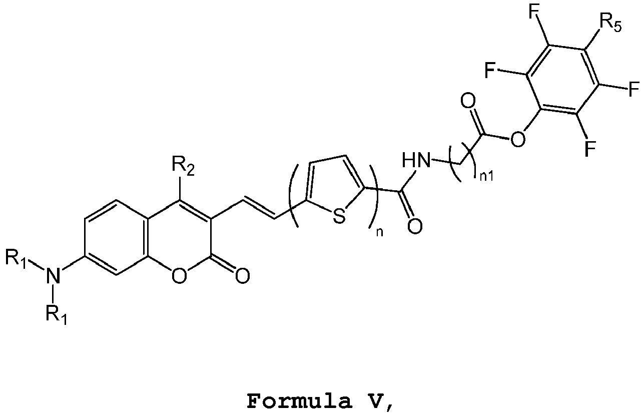 wherein:
   - n: is 1 or 2 n₁ is, 1, 2, 3, 4 or 5
   - R₁: is -(CH2)n-CH3, wherein n is 0, 1, 2 or 3; and
   - R₂: is -(CH₂)ₙ-CH₃, and n is 0 or 1.
p) **reacting compounds of formula (XII)** with methanediamine (or ethane-1,2-diamine, or propane-1,3-diamine, or butane-1,4-diamine, or pentane-1,5-diamine), and N,N'-dicyclohexylcarbodiimide in a reaction solvent selected from the group comprising acetonitrile and N,N-dimethylformamide, or dimethyl sulfoxide, a mixed solvent thereof and the like, and stirring at a temperature between 20 °C and 26 °C for 24 hours to 72 hours; and
   evaporating the reaction mixture to dryness and purifying the residue to **produce compounds of formula (XVIII).** 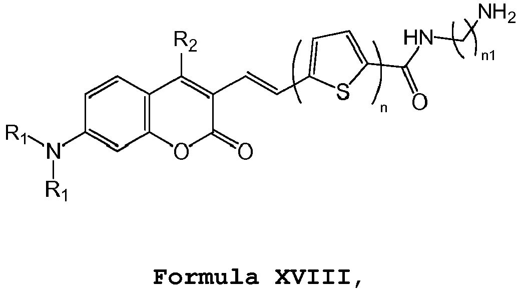 wherein:
   - n: is 1 or 2
   - n₁: is, 1, 2, 3, 4 or 5
   - R₁: is -(CH2)n-CH3, wherein n is 0, 1, 2 or 3; and
   - R₂: is -(CH₂)ₙ-CH₃, and n is 0 or 1.
q) **reacting compounds of formula (XVIII)** with maleic anhydride, in a reaction solvent selected from the group comprising acetonitrile, or N,N-dimethylformamide, or dimethyl sulfoxide, a mixed solvent thereof and the like, and stirring at a temperature between 20 °C and 26 °C for 3 hours to 24 hours; and
   evaporating the reaction mixture to dryness and purifying the residue to **produce compounds of formula (XIX).** 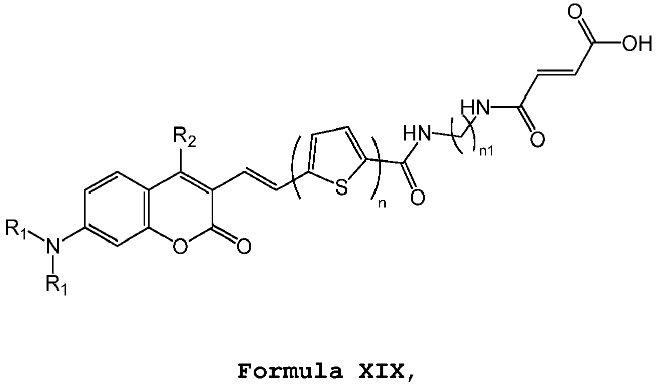 wherein:
   - n: is 1 or 2
   - n₁: is, 1, 2, 3, 4 or 5
   - R₁: is -(CH2)n-CH3, wherein n is 0, 1, 2 or 3; and
   - R₂: is -(CH₂)ₙ-CH₃, and n is 0 or 1.
r) **reacting compounds of formula (XIX)** with acetic anhydride and triethylamine, in a reaction solvent selected from the group comprising acetonitrile, or N,N-dimethylformamide, or dimethyl sulfoxide, a mixed solvent thereof and the like, and stirring at a temperature between 60 °C and 100 °C for 24 hours to 72 hours; and
   evaporating the reaction mixture to dryness and purifying the residue to **produce compounds of formula (XX).** 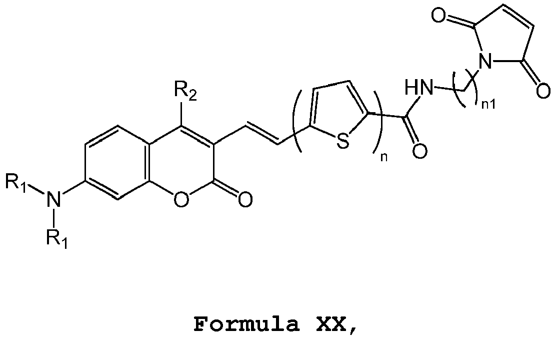 wherein:
   - n: is 1 or 2
   - n₁: is, 1, 2, 3, 4 or 5
   - R₁: is -(CH2)n-CH3, wherein n is 0, 1, 2 or 3; and
   - R₂: is -(CH₂)ₙ-CH₃, and n is 0 or 1.
s) **reacting compounds of formula (XX)** with fuming sulfuric acid (30% SO3), and stirring at a temperature between 20 °C and 26 °C for 30 minutes to 1 hour; and adding NaCl until no further precipitate formation; and
   recrystallizing the reaction mixture in acetonitrile to **produce compounds of formula (VI).** 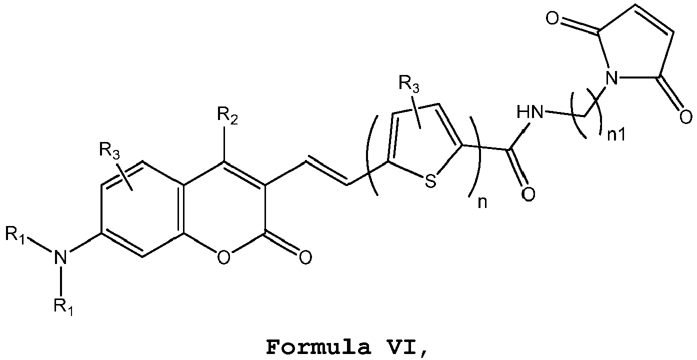 wherein:
   - n: is 1 or 2
   - n₁: is, 1, 2, 3, 4 or 5
   - R₁: is -(CH2)n-CH3, wherein n is 0, 1, 2 or 3;
   - R₂: is -(CH₂)ₙ-CH₃, and n is 0 or 1; and
   - R₃: is independently H or SO₃⁻ Na⁺.

Therefore, compounds of formula I, in particular compounds of formulae (1), (2), (3), (4) and (5) **are useful as intermediaries** to produce new **fluorescent labels for biomolecules of formulae** (II), (III), (IV), (V), (VI) as below: 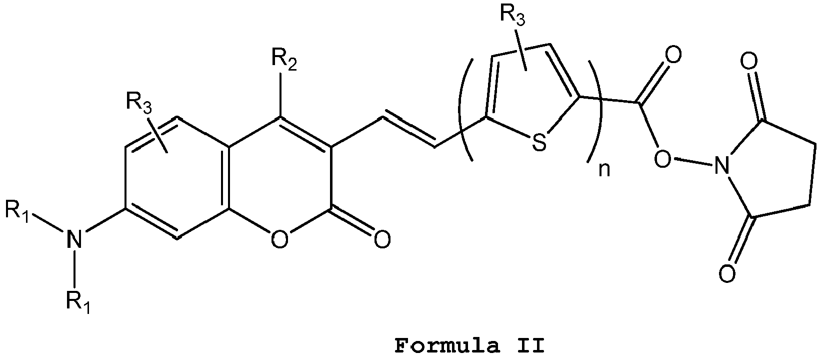 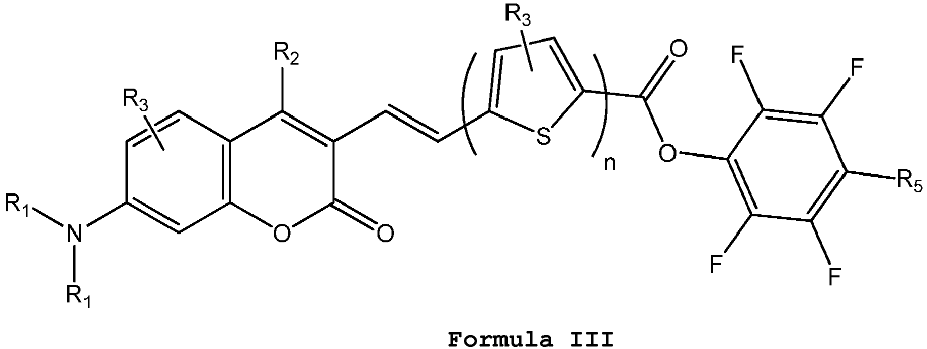 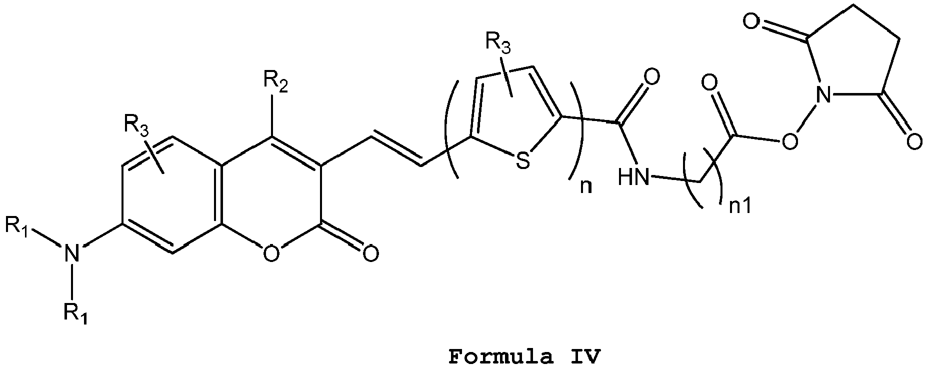 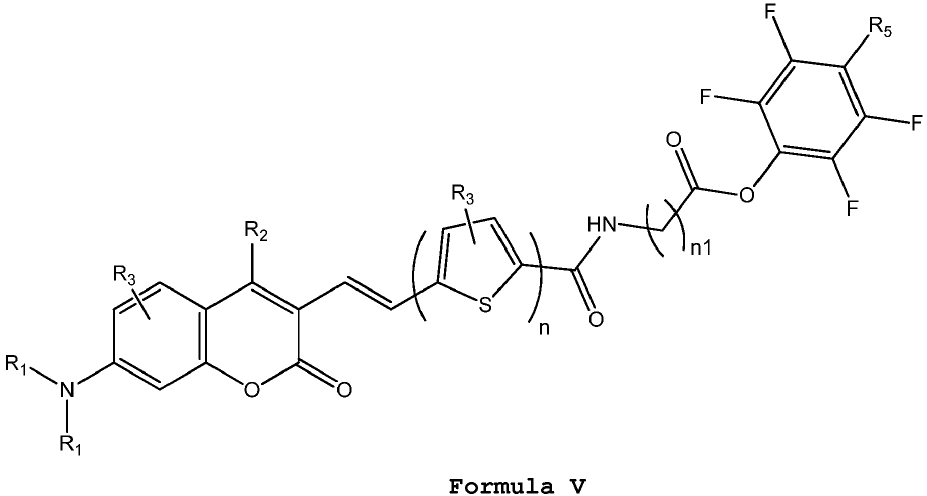 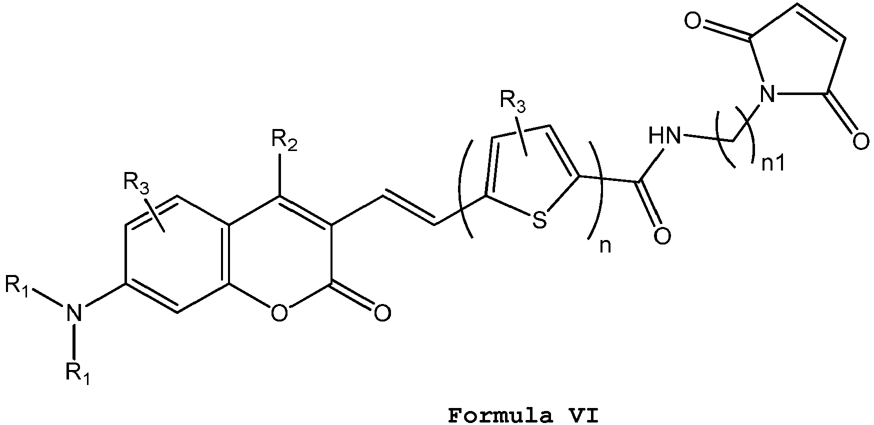 wherein:
- n: is 1 or 2,
- n₁: is 1, 2, 3, 4 or 5,
- R₁: is -(CH₂)ₙ-CH₃, and n is 0, 1, 2 or 3,
- R₂: is -(CH₂)ₙ-CH₃, and n is 0 or 1,
- R₃: is independently H or SO₃⁻ Na⁺,
- R₅: is H, F or SO₃⁻ Na⁺

### 2. Method for producing coumarin compounds derivatives of formula and (VII)

Further **dye-sensitized solar cells (DSSCs)** compounds are obtained by a process comprising the following steps:
a) **reacting a compound of formula (X)** with 5-bromothiophene-2-carbaldehyde (or 5'-bromo-[2,2'-bithiophene]-5-carbaldehyde), tetrakis(triphenylphosphine)palladium(0) and silver acetate in a reaction solvent selected from the group comprising N,N-dimethylformamide, dimethyl sulfoxide, a mixed solvent thereof and the like, and stirring under inert atmosphere at a temperature between 78 °C and 98 °C for 24 hours to 72 hours; and evaporating the reaction mixture to dryness and purifying the residue to **produce compounds of formula (XXI).** 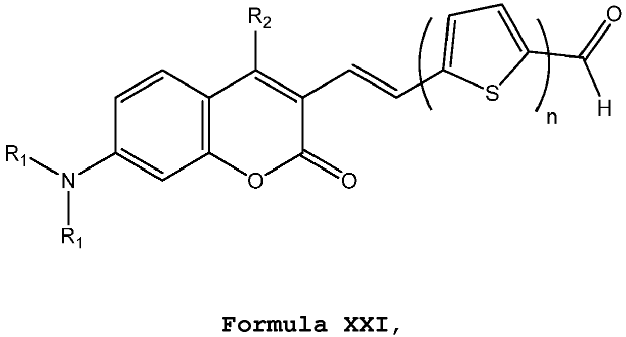 Wherein:
   - n=1: or 2
   - R₁: is -(CH₂)ₙ-CH₃, and n is 0, 1, 2 or 3,
   - R₂: is -(CH₂)ₙ-CH₃, and n is 0 or 1

Therefore, compounds (19) are useful as intermediary compounds for producing dye-sensitized solar cells compounds of formulae (20) as below:
b) **reacting compounds of formula (XXI)** with cyanoacetic acid and piperidine in a reaction solvent selected from the group comprising acetonitrile or N,N-dimethylformamide, or dimethyl sulfoxide, a mixed solvent thereof and the like, and stirring at a temperature between 70 °C and 100 °C for 1 hour to 6 hours; and
evaporating the reaction mixture to dryness and purifying the residue to **produce compounds of formula (VII).** 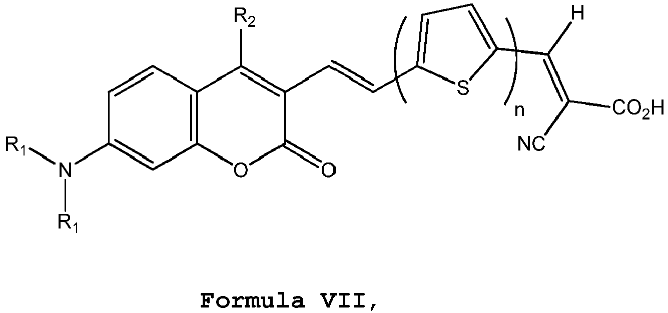 Wherein:
- n=1: or 2
- R₁: is -(CH₂)ₙ-CH₃, and n is 0, 1, 2 or 3,
- R₂: is -(CH₂)ₙ-CH₃, and n is 0 or 1,

These dyes are water soluble and can be excited by the 435 nm excitation source and exhibit a large Stokes shift (≥96 nm).

Furthermore, the dyes possess a reactive group for the labelling of biomolecules or other analytes, and a group to anchor on the surface of semiconducting TiO₂ nanocrystals. The process of the present invention is based on specific chromophores such as the ones of the general formula I and their derivatives. Compounds obtained as described above, namely compounds of formulae (1), (3), (4), (6), (7), (8), and (9) produce spectra of Fig.1, and Fig.3 (UV-Abs), Fig.2 and Fig.4 (UV-Em) respectively. Characteristics of these compounds are listed in Table 1 and in Table 2. Compounds of Table 1 are also listed in Table 2 for easier comparison of their characteristics.

**Table 1**

| **Compound** | **λ_{abs} (nm) (****Fig. 1****)** | **λₑₘ (nm) (****Fig.2****)** |
|---|---|---|
| 7-diethylamino-4-methylcoumarin (1) | 371 | 434 |
| 7-diethylamino-4-methyl-3-vinylcoumarin (3) | 388 | 461 |
| (*E*)-methyl 5-(2-(7-(diethylamino)-4-methyl-coumarin-3-yl)vinyl)-thiophene-2-carboxylate (4) | 435 | 531 |
| sodium (*E*/*Z*)-4-((5-(2-(7-(diethylamino)-4-methyl-coumarin-3-yl)vinyl)thiophene-2-carbonyl)oxy)-2,3,5,6-tetrafluorobenzenesulfonate (6) | 457 | 569 |

**Table 2**

| **Compound** | **λ_{abs} (nm) (****Fig.3****)** | **λₑₘ (nm) (****Fig.4****)** |
|---|---|---|
| 7-diethylamino-4-methylcoumarin (1) | 371 | 434 |
| 7-diethylamino-4-methyl-3-vinylcoumarin (3) | 388 | 461 |
| (*E*)-5-(2-(7-(diethylamino)-4-methyl-coumarin-3-yl)vinyl)thiophene-2-carbaldehyde (7) | 446 | 576 |
| (*E*)-5'-(2-(7-(diethylamino)-4-methyl-coumarin-3-yl)vinyl)-[2,2'-bithiophene]-5-carbaldehyde (8) | 464 | 599 |
| (*E*)-2-cyano-3-(5-((*E*)-2-(7-(diethylamino)-4-methyl-coumarin-3-yl)vinyl)thiophen-2-yl)acrylic acid (9) | 496 | 569 |

The chromophores of the present invention selectively bind to a specific region or functional group on the target biomolecule to attach chemically to them.

### EXAMPLES

### Example 1. Synthesis of sodium (E)-4-((5-(2-(7-(diethylamino)-4-methyl-coumarin-3-yl)vinyl)thiophene-2-carbonyl)oxy)-2,3,5,6-tetrafluorobenzenesulfonate.

### 1.1 Preparation of 3-bromo-7-diethylamino-4-methyl-coumarin

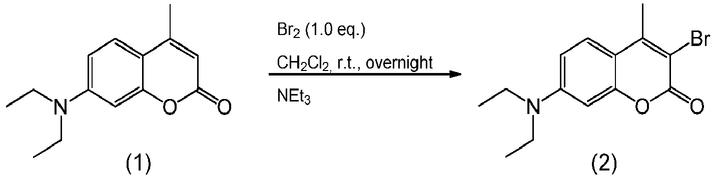

To a solution of 7-diethylamino-4-methyl-coumarin (1)(100 mg, 0.432 mmol) in dichloromethane (2 mL) was added bromine (0.022 mL, 0.432 mmol) in an ice bath. After stirring overnight at room temperature, NEt₃ (0.200 mL) was added cautiously. After stirring for further 30 minutes, the reaction mixture was evaporated to dryness and the residue was purified by flash column chromatography on silica gel (230-400 mesh; CHCl₃/MeOH (99:1)) to yield 3-bromo-7-diethylamino-4-methyl-coumarin (2) (131 mg, 0.424 mmol, 98%).
¹H NMR (400 MHz, CDCl₃) δ (ppm): 1.19 (6H, t, *J* = 7.1, N(CH₂*CH*₃)₂), 2.50 (3H, s, 4-CH₃), 3.40 (4H, q, *J* = 7.1, N(*CH₂*CH₃)₂), 6.46 (1H, s, H-8), 6.59 (1H, d, *J*_{6,5} = 9.0, H-6), 7.40 (1H, d, *J*₅,₆= 9.0, H-5) .
¹³C NMR (100 MHz, CDCl₃) δ (ppm) : 12.5 (C11, C13), 19.2 (C9), 44.9 (C10, C12), 97.3 (C8), 105.7 (C3), 109.0 (C4a), 126.2 (C6), 126.2 (C5), 150.7 (C7), 151.6 (C4), 154.5 (C8a), 158.3 (C2).

### 1.2 Preparation of 7-diethylamino-4-methyl-3-vinylcoumarin.

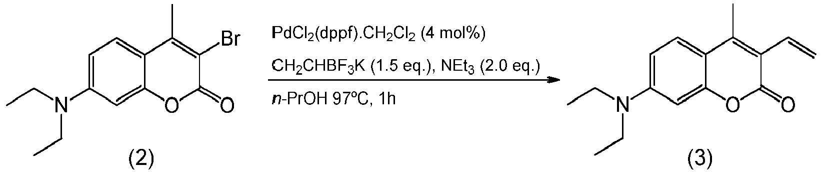

A mixture of 3-bromo-7-(diethylamino)-4-methylcoumarin (2) (100 mg, 0.322 mmol), potassium vinyltrifluoroborate (65 mg, 0.484 mmol), PdCl₂ (dppf) .CH₂Cl₂ (10 mg, 0.013 mmol), and NEt₃ (0.090 mL, 0.645 mmol) in n-PrOH (6 mL) was stirred at reflux under inert atmosphere for a period of 1 h. After cooling to room temperature, the reaction mixture was evaporated to dryness and the residue was purified by flash column chromatography on silica gel (230-400 mesh; CH₂Cl₂) to yield 7-diethylamino-4-methyl-3-vinyl coumarin (3) (80 mg, 0.309 mmol, 96%).
¹H NMR (400 MHz, CDCl₃) δ (ppm) : 1.19 (6H, t, *J* = 7.1, N(CH₂*CH₃*)₂)*,* 2.41 (3H, s, 4-CH₃), 3.39 (4H, q, *J* = 7.1, N(*CH₂*CH₃)₂)*,* 5.51 (1H, dd, *J*_{2'a, 2'b} = 2.08, 2.1, *J*_{2*'*a, 1'} = 11.7, H-2'a), 6.03 (1H, dd, *J*_{2'b, 2'a} = 2.1, J_{2'b, 1'} = 17.5, H-2'b), 6.46 (1H, d, *J*_{8,6}= 2.6, H-8), 6.58 (1H, dd, *J*_{6,8} = 2.6, *J*_{6,5} = 9.1, H-6), 6.70 (1H, dd, *J*_{1',2'a} = 11.7, *J*_{1',2'b} = 17.5, H-1'), 7.43 (1H, d, *J*_{5,6}= 9.1, H-5).
¹³C NMR (100 MHz, CDCl₃) δ (ppm) : 12.7 (C11, C13), 15.1 (C9), 44.9 (C10, C12), 97.3 (C8), 108.8 (C6), 109.7 (C3), 116.4 (C4a), 120.3 (C2'), 126.2 (C5), 129.5 (C1'), 147.7 (C7), 150.3 (C4), 154.6 (C8a), 161.2 (C2).
MS-TOF (+) calc. for C₁₆H₁₉NO₂Na [M+Na]⁺ 280.1308 found 280.1309 IR V^{max} (cm⁻¹) : 2969, 2926, 1703, 1620, 1604, 1577, 1517, 1409, 1355, 1263, 1145, 1076, 823, 786.
UV λ^{max} (nm, CH₃CN) : 208, 261, 388.

### Example 2. Preparation of (E)-methyl 5-(2-(7-(diethylamino)-4-methyl-coumarin-3-yl)vinyl)thiophene-2-carboxylate.

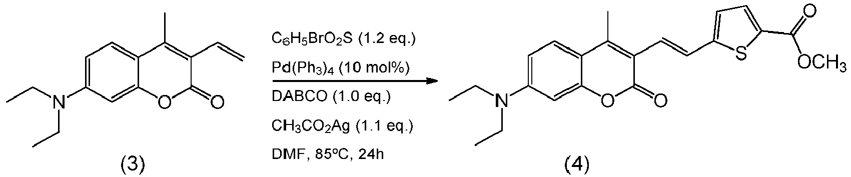

A mixture of 7-diethylamino-4-methyl-3-vinyl coumarin (3) (100 mg, 0.389 mmol), methyl 5-bromothiophene-2-carboxylate (103 mg, 0.466 mmol), Pd(PPh₃)₄ (45 mg, 0.039 mmol), DABCO (44 mg, 0.389 mmol) and CH₃CO₂Ag (71 mg, 0.427 mmol) in DMF (2.0 mL) was stirred at 85°C, under inert atmosphere, for a period of 24 h. After cooling to room temperature, the reaction mixture was evaporated to dryness and the residue was purified by flash column chromatography on silica gel (230-400 mesh; CHCl₃) to yield ((*E*)-methyl 5-(2-(7-(diethylamino)-4-methyl-coumarin-3-yl)vinyl)thiophene-2-carboxylate (4) (96 mg, 0.241 mmol, 62%).
¹H NMR (400 MHz, CDCl₃) δ (ppm): 1.21 (6H, t, *J* = 7.0, N(CH₂*CH₃*)₂)*,* 2.48 (3H, s, 4-CH₃), 3.42 (4H, q, *J* = 7.0, N(*CH₂*CH₃)₂)*,* 3.88 (3H, s, OCH₃), 6.48 (1H, d, *J₈*,₆ = 2.4, H-8), 6.61 (1H, dd, *J*_{6,5} = 2.4, *J*_{6,5} = 9.2, H-6), 7.03 (1H, d, *J*_{4',5'} = 4.0, H-4'), 7.13 (1H, d, *J*_{1', 2'} = 16.0, H-1'), 7.49 (1H, d, *J*_{5,6}= 9.2, H-5), 7.67 (1H, d, *J*_{5',4'} = 4.0, H-5'), 7.97 (1H, d, *J*_{2', 1'} = 16.0, H-2').
¹³C NMR (100 MHz, CDCl₃) δ (ppm) : 12.6 (C11, C13), 14.8 (C9), 44.9 (C10, C12), 52.3 (OCH₃), 97.3 (C8), 109.1 (C6), 109.8 (C4a), 114.4 (C3), 124.0 (C1'), 125.6 (C2'), 126.5 (C5), 126.6 (C4'), 130.8 (C6'), 134.4 (C5'), 148.7 (C4), 150.6 (C7), 151.4 (C3'), 154.8 (C8a), 160.6 (C2), 162.8 (*COO*).
MS-TOF (+) calc. for C₂₂H₂₄NO₄S [M+H]⁺ 398.1420 found 398.1417; calc. for C₂₂H₂₃NO₄NaS [M+Na]⁺ 420.1246 found 420.1230. IR V^{max} (cm⁻¹) : 2970, 2925, 1705, 1618, 1596, 1570, 1527, 1511, 1437, 1413, 1378, 1354, 1288, 1264, 1147, 1097, 951, 824, 766, 748.
UV λ^{max} (nm, CH₃CN) : 252, 316, 435.

### Example 3. Preparation of (E)-5-(2-(7-(diethylamino)-4-methyl-coumarin-3-yl)vinyl)thiophene-2-carboxylic acid.

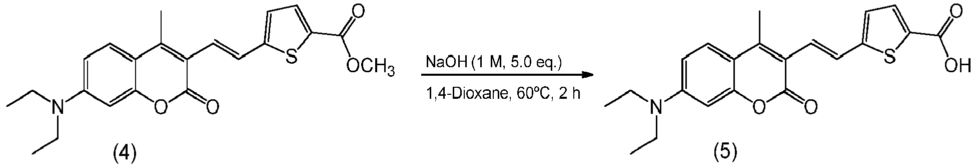

A mixture of ((*E*)-methyl 5-(2-(7-(diethylamino)-4-methyl-coumarin-3-yl)vinyl)thiophene-2-carboxylate (4) (100 mg, 0.252 mmol) in 1,4-dioxane (1.3 mL) was added 1 M sodium hydroxide (1.3 mL, 1.26 mmol) was stirred at 60°C for about 2 h. After cooling to room temperature, the reaction it was neutralized with HCl (10%) solution. The reaction mixture was evaporated to dryness and the residue was purified by flash column chromatography on silica gel (230-400 mesh; CHCl₃/MeOH (9:1) to yield (*E*)-5-(2-(7-(diethylamino)-4-methyl-coumarin-3-yl)vinyl)thiophene-2-carboxylic acid (5) (87 mg, 0.226 mmol, 90%) .
¹H NMR (400 MHz, DMSO-d₆) δ (ppm) : 1.13 (6H, t, *J* = 7.0, N(CH₂*CH₃*)₂)*,* 2.51 (3H, s, 4-CH₃), 3.44 (4H, q, *J* = 7.0, N(*CH₂*CH₃)₂), 6.51 (1H, d, *J*_{8,6} = 2.2, H-8), 6.72 (1H, dd, *J*_{6,8} = 2.2, *J*_{6,5} = 9.2, H-6), 7.14 (1H, d, *J*_{1',2'} = 15.8, H-1'), 7.27 (1H, d, *J*_{4',5'} = 3.8, H-4'), 7.62 (1H, d, *J*_{5',4'} = 3.8, H-5'), 7.66 (1H, d, *J*_{5,6}= 9.2, H-5), 7.83 (1H, d, *J*_{2',1'} = 15.8, H-2').
¹³C NMR (100 MHz, DMSO-d₆) δ (ppm): 12.4 (C11, C13), 14.6 (C9), 44.1 (C10, C12), 96.2 (C8), 108.9 (C6), 109.3 (C4a), 112.8 (C3), 124.0 (C2'), 124.2 (C1'), 126.6 (C5), 127.2 (C4'), 132.5 (C6'), 133.9 (C5') 149.7 (C4), 149.8 (C7), 150.5 (C3'), 154.3 (C8a), 159.4 (C2), 162.9 (*C*O₂H).
MS-TOF (+) calc. for C₂₁H₂₁NO₄NaS [M+Na]⁺ 406.1083 found 406.1086.
IR V^{max} (cm⁻¹) : 3388, 2968, 2925, 1698, 1614, 1568, 1519, 1441, 1414, 1356, 1264, 1143, 1074, 1026, 951.

### Example 4. Preparation of sodium (E/Z)-4-((5-(2-(7-(diethylamino)-4-methyl-coumarin-3-yl)vinyl)thiophene-2-carbonyl)oxy)-2,3,5,6-tetrafluorobenzenesulfonate

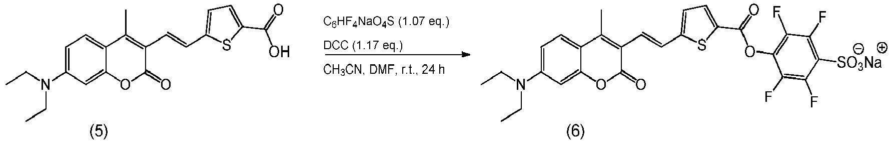

A mixture of (*E*)-5-(2-(7-(diethylamino)-4-methyl-coumarin-3-yl)vinyl)thiophene-2-carboxylic acid (5) (100 mg, 0.261 mmol), 4-sulfotetrafluorophenol sodium salt (75 mg, 0.279 mmol) and *N,N'*-dicyclohexylcarbodiimide (63 mg, 0.305 mmol) in CH₃CN (*4* mL) and DMF (1 mL) was stirred at room temperature for a period of 24 h. The reaction mixture was evaporated to dryness and the residue was purified by flash column chromatography on silica gel (230-400 mesh; CHCl₃/MeOH/H₂O (65:20:2) to yield sodium (E/Z)-4-((5-(2-(7-(diethylamino)-4-methyl-coumarin-3-yl)vinyl)thiophene-2-carbonyl)oxy)-2,3,5,6-tetrafluorobenzenesulfonate (6) (144 mg, 0.227 mmol, 87%).

### E-isomer (53%)

¹H NMR (400 MHz, DMSO-d₆) δ (ppm) : 1.10-1.14 (6H, t, N(CH₂*CH₃*)₂)*,* 2.55 (3H, s, 4-CH₃), 3.40-3.45 (4H, q, N(*CH₂*CH₃)₂), 6.49-6.58 (1H, m, H-8), 6.68-6.78 (1H, m, H-6), 7.34 (1H, d, *J*_{1'*,*2'} = 16.0, H-1'), 7.50 (1H, d, *J*_{4',5'} = 4.0, H-4'), 7.69 (1H, d, *J*_{5,6}= 9.2, H-5), 7.96 (1H, d, *J*_{2',1'} = 16.0, H-2'), 8.09 (1H, d, *J*_{5',4'} = 4.0, H-5').
¹³C NMR (100 MHz, DMSO-d₆) δ (ppm): 12.4 (C11, C13), 14.6 (C9), 44.1 (C10, C12), 96.2 (C8), 108.9 (C6), 109.0 (C4a), 112.4 (C3), 123.1 (C2'), 124.9 (C6'), 126.4 (C1'), 127.4 (C4'), 127.5 (C5), 127.7-128.0 (C11'), 138.4 (C5'), 138.8-139.0 (C10', C12'), 141.4-141.8 (C8'), 144.1-144.4 (C9', C13'), 150.8 (C4), 151.1 (C3'), 154.5 (C8a), 155.0 (C7), 157.4 (COO), 159.4 (C2).

### Z-isomer (47%)

¹H NMR (400 MHz, DMSO-d₆) δ (ppm): 1.10-1.14 (6H, t, N(CH₂*CH₃*)₂)*,* 2.26 (3H, s, 4-CH₃), 3.40-3.45 (4H, q, N(*CH₂*CH₃)₂), 6.49-6.58 (2H, m, H-8, H1'), 6.68-6.78 (1H, m, H-6), 7.14 (1H, d, *J*_{2',1'} = 11.6, H-2'), 7.36 (1H, d, *J*_{4',5'} = 4.0, H-4'), 7.58 (1H, d, *J*_{5,6}= 9.1, H-5), 8.05 (1H, d, *J*_{5',4'} = 4.0, H-5').
¹³C NMR (100 MHz, DMSO-d₆) δ (ppm) : 12.4 (C11, C13), 15.2 (C9), 44.0 (C10, C12), 96.5 (C8), 108.3 (C4a), 109.4 (C6), 114.1 (C3), 125.5 (C6'), 126.1 (C2'), 127.0 (C5), 127.2 (C1'), 127.7-128.0 (C11'), 130.8 (C4'), 137.1 (C5'), 138.8-139.0 (C10', C12'), 141.4-141.8 (C8'), 144.1-144.4 (C9', C13'), 149.5 (C3'), 150.6 (C4), 154.5 (C8a), 155.0 (C7), 157.5 (CO₂H), 159.2 (C2).
MS-TOF (+) calc. for C₂₇H₂₀F₄NO₄NaO₇S₂ [M+Na]⁺ 656.0407 found 656.0410.
IR V^{max} (cm⁻¹): 2977, 2922, 2855, 1741, 1708, 1616, 1491, 1424, 1357, 1247, 1096, 1049, 988.
UV λ^{max} (nm, CH₃CN) : 255, 324, 408, 457.

### Example 5. Synthesis of (E)-2-cyano-3-(5-((E)-2-(7-(diethylamino)-4-methyl-coumarin-3-yl)vinyl)thiophen-2-yl)acrylic acid.

### 5.1 Preparation of (E)-5-(2-(7-(diethylamino)-4-methyl-coumarin-3-yl)vinyl)thiophene-2-carbaldehyde.

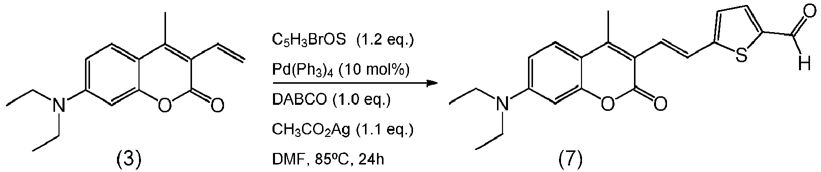

A mixture of 7-diethylamino-4-methyl-3-vinyl coumarin (3) (100 mg, 0.389 mmol), 5-bromothiophene-2-carbaldehyde (89 mg, 0.466 mmol), Pd(PPh₃)₄ (45 mg, 0.039 mmol), DABCO (44 mg, 0.389 mmol) and CH₃CO₂Ag (71 mg, 0.427 mmol) in DMF (2.0 mL) was stirred at 85°C, under inert atmosphere, for a period of 24 h. After cooling to room temperature, the reaction mixture was evaporated to dryness and the residue was purified by flash column chromatography on silica gel (230-400 mesh; CHCl₃) to yield (*E*)-5-(2-(7-(diethylamino)-4-methyl-coumarin-3-yl)vinyl)thiophene-2-carbaldehyde (7) (93 mg, 0.253 mmol, 65%).
¹H NMR (400 MHz, CDCl₃) δ (ppm): 1.21 (6H, t, *J* = 7.1, N(CH₂*CH₃*)₂)*,* 2.50 (3H, s, 4-CH₃), 3.42 (4H, q, *J* = 7.1, N(*CH₂*CH₃)₂), 6.47 (1H, d, *J*_{8,6} = 2.5, H-8), 6.61 (1H, dd, *J*_{6,8} = 2.5, *J*_{6,5} = 9.1, H-6), 7.13 (1H, d, *J*_{4',5'} = 3.9, H-4'), 7.22 (1H, d, *J*_{1',2'} = 15.6, H-1'), 7.50 (1H, d, *J*_{5,6}= 9.1, H-5), 7.64 (1H, d, *J*_{5',4'} = 3.9, H-5'), 8.04 (1H, d, *J*_{2',1'} = 15.6, H-2'), 9.82 (1H, s, CHO).
¹³C NMR (100 MHz, CDCl₃) δ (ppm): 12.6 (C11, C13), 14.8 (C9), 45.0 (C10,C12), 97.2 (C8), 109.2 (C6), 109.7 (C4a), 114.0 (C3), 125.1 (C1'), 125.6 (C2'), 126.6 (C5), 127.1 (C4'), 137.7 (C5'), 141.2 (C6'), 149.5 (C4), 150.8 (C7), 154.5 (C3'), 154.9 (C8a), 160.5 (C2), 182.7 (CHO).
MS-TOF(+) calc. for C₂₁H₂₁NNaO₃S [M+Na]⁺ 390.1134 found 390.1141.
IR V^{max} (cm⁻¹): 2973, 2923, 1707, 1658, 1615, 1592, 1516, 1423, 1353, 1264, 1224, 1145, 1076, 1047, 1047, 951, 797, 765.
UV λ^{max} (nm, CH₃CN): 257, 332, 402, 446.

### 5.2 Preparation of (E)-5'-(2-(7-(diethylamino)-4-methyl-coumarin-3-yl)vinyl)-[2,2'-bithiophene]-5-carbaldehyde.

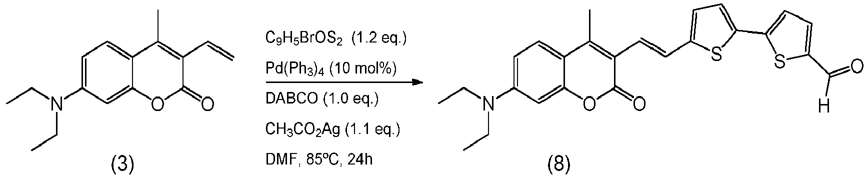

A mixture of 7-diethylamino-4-methyl-3-vinyl coumarin (3) (100 mg, 0.389 mmol), 5'-bromo-[2,2'-bithiophene]-5-carbaldehyde (127 mg, 0.466 mmol), Pd(PPh₃)₄ (45 mg, 0.039 mmol), DABCO (44 mg, 0.389 mmol) and CH₃CO₂Ag (71 mg, 0.427 mmol) in DMF (2.0 mL) was stirred at 85°C, under inert atmosphere, for a period of 24 h. After cooling to room temperature, the reaction mixture was evaporated to dryness and the residue was purified by flash column chromatography on silica gel (230-400 mesh; CHCl₃) to yield (*E*)-5'-(2-(7-(diethylamino)-4-methyl-coumarin-3-yl)vinyl)-[2,2'-bithiophene]-5-carbaldehyde (8) (122 mg, 0.272 mmol, 70 %).
¹H NMR (400 MHz, CDCl₃) δ (ppm): 1.23 (6H, t, *J* = 7.0, N(CH₂*CH₃*)₂)*,* 2.52 (3H, s, 4-CH₃), 3.43 (4H, q, *J* = 7.0, N(*CH₂*CH₃)₂)*,* 6.52 (1H, s, H-8), 6.66 (1H, d, *J*₆,₅ = 8.8, H-6), 7.03 (1H, d, *J*_{1',2'} = 15.6, H-1'), 7.03 (1H, d, *J*_{4',5'} = 3.6, H-4'), 7.24 (1H, d, *J*_{8',9'} = 4.0, H-8'), 7.26 (1H, d, *J*_{5',4'} = 3.6, H-5'), 7.51 (1H, d, *J*_{5,6}= 9.2, H-5), 7.66 (1H, d, *J*_{9',8'} = 4.0, H-9'), 7.97 (1H, d, *J_{2'},*_{1'} = 15.6, H-2'), 9.85 (1H, s, CHO).
¹³C NMR (100 MHz, CDCl₃) δ (ppm): 12.6 (C11, C13), 14.9 (C9), 45.2 (C10, C12), 97.6 (C8), 109.4 (C6), 115.0 (C3), 115.2 (C4a), 122.6 (C1'), 124.1 (C8'), 125.9 (C2'), 126.4 (C5), 127.1 (C5'), 127.9 (C4'), 134.3 (C6'), 137.6 (C9'), 141.5 (C10'), 146.5 (C3'), 147.5 (C7'), 147.8 (C4), 150.2 (C7), 154.7 (C8a), 160.6 (C2), 182.5 (CHO).
MS-TOF (+) calc. for C₂₅H₂₃NO₃S₂ [M]⁺ 449.1119 found 449.1085; calc. for C₂₅H₂₄NO₃S₂ [M+H]⁺ 450.1192 found 450.1181.
IR V^{max} (cm⁻¹) : 2918, 2850, 1699, 1652, 1615, 1593, 1567, 1514, 1441, 1412, 1381, 1355, 1263, 1225, 1049, 959, 816, 789, 764. UV λ^{max} (nm, CH₃CN) : 258, 464.

### 5.3 Preparation of E-2-cyano-3-(5-((E)-2-(7-(diethylamino)-4-methyl-coumarin-3-yl)vinyl)thiophen-2-yl)acrylic acid.

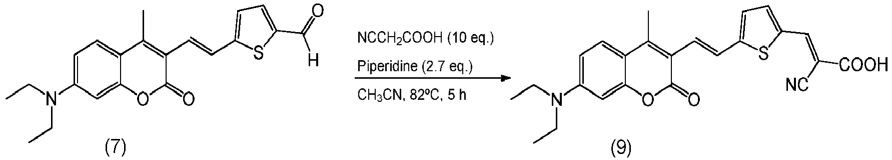

A mixture of yield (*E*)-5-(2-(7-(diethylamino)-4-methyl-coumarin-3-yl)vinyl)thiophene-2-carbaldehyde (7) (100 mg, 0.272 mmol), cyanoacetic acid (231 mg, 2.72 mmol) and piperidine (0.073 mL, 0.735 mmol) in CH₃CN (12 mL) was stirred at 82°C for a period of 5 h. After cooling to room temperature., the reaction mixture was evaporated to dryness and the residue was purified by flash column chromatography on silica gel (230-400 mesh; CHCl_{3/}MeOH/H₂O (65:35:5) to yield *E*-2-cyano-3-(5-((*E*)-2-(7-(diethylamino)-4-methyl-coumarin-3-yl)vinyl)thiophen-2-yl)acrylic acid (9) (109 mg, 0.251 mmol, 92 %) .
¹H NMR (400 MHz, DMSO-d₆) δ (ppm) : 1.12 (6H, t, *J* = 7.0, N(CH₂*CH₃*)₂)*,* 2.50 (3H, s, 4-CH₃), 3.43 (4H, q, *J* = 7.0, N(*CH₂*CH₃)₂), 6.49 (1H, d, *J*_{8*,*6} = 2.4, H-8), 6.71 (1H, dd, *J*_{6,8} = 2.4, *J*_{6,5} = 9.2, H-6), 7.11 (1H, d, *J*_{1',2'} = 15.6, H-1'), 7.34 (1H, d, *J*_{4',5'} = 4.0, H-4'), 7.64 (1H, d, *J*_{5,6}= 9.2, H-5), 7.66 (1H, d, *J*_{5',4'} = 4.0, H-5'), 7.84 (1H, d, *J*_{2',1'} = 15.6, H-2'), 8.12 (1H, s, H-7').
¹³C NMR (100 MHz, DMSO-d₆) δ (ppm): 12.4 (C11, C13), 14.6 (C9), 44.1 (C10,C12), 96.2 (C8), 108.9 (C6), 109.3 (C3, C4a), 112.9 (C8'), 118.9 (CN), 124.1 (C1'), 124.2 (C2'), 126.9 (C4'), 127.2 (C5), 135.3 (C6'), 136.7 (C5'), 141.3 (C7'), 149.3 (C4), 149.6 (C7), 150.5 (C3'), 154.3 (C8a), 159.4 (C2), 163.7 (CO₂H).
MS-TOF(+) calc. for C₂₄H₂₂N₂NaO₄S [M+Na]⁺ 457.1192 found 457.1195.
IR V^{max} (cm⁻¹) : 3401, 2973, 2212, 1705, 1615, 1569, 1519, 1377, 1356, 1264, 1214, 1147, 1079, 949, 800, 766.
UV λ^{max} (nm, CH₃CN) : 378, 496.

## Claims

**1.** Fluorescent vinyl tiophene and/or bitiophene coumarins, in particular 7-alkylamino-3-vinyl tiophene and/or 7-alkylamino-3-vinyl ditiophene coumarin of general formula (I) : 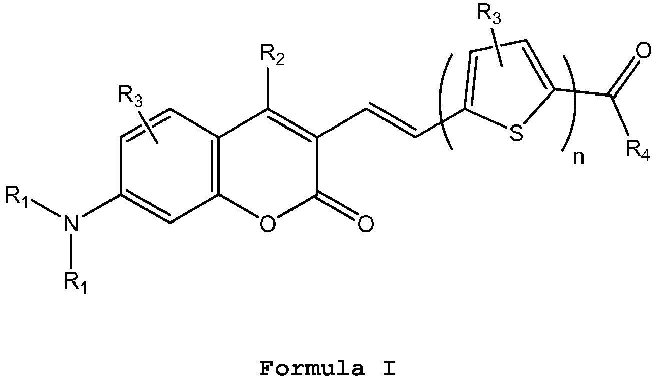 wherein:
n is 1 or 2,
R₁ and R₂ are independently -(CH₂)ₙ-CH₃, and n is 0, 1, 2 or 3;
R₃ is independently H or SO₃⁻ Na⁺; and
R₄ is H or OCH₃.

**2.** Fluorescent coumarins according to claim 1 having the general formula (II), (III), (IV), (V), (VI) and (VII): 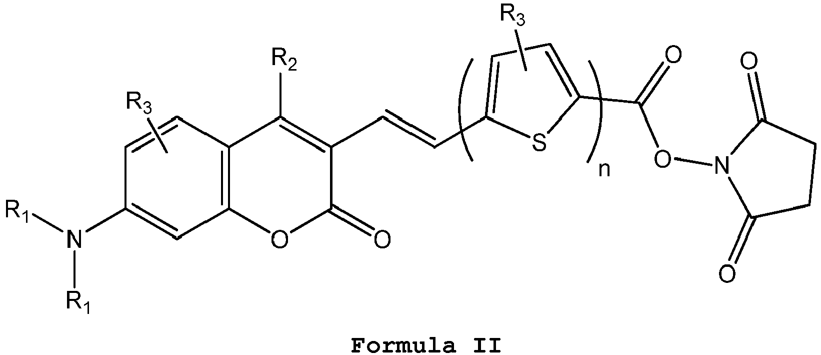 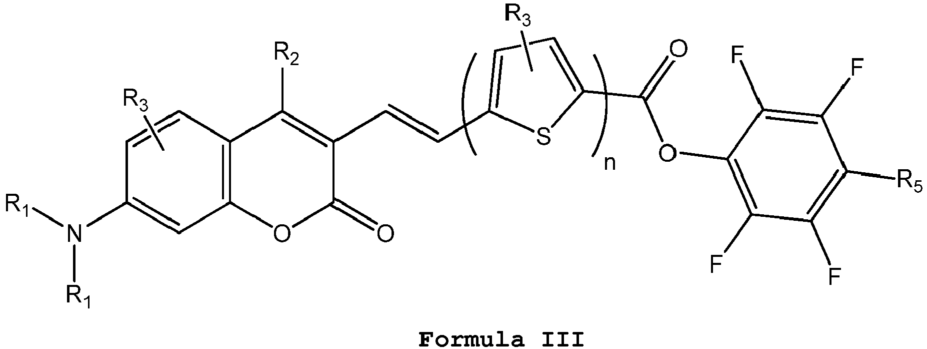 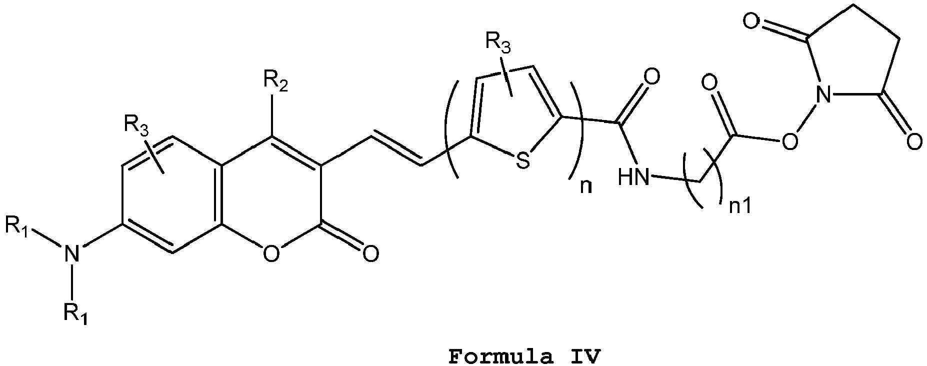 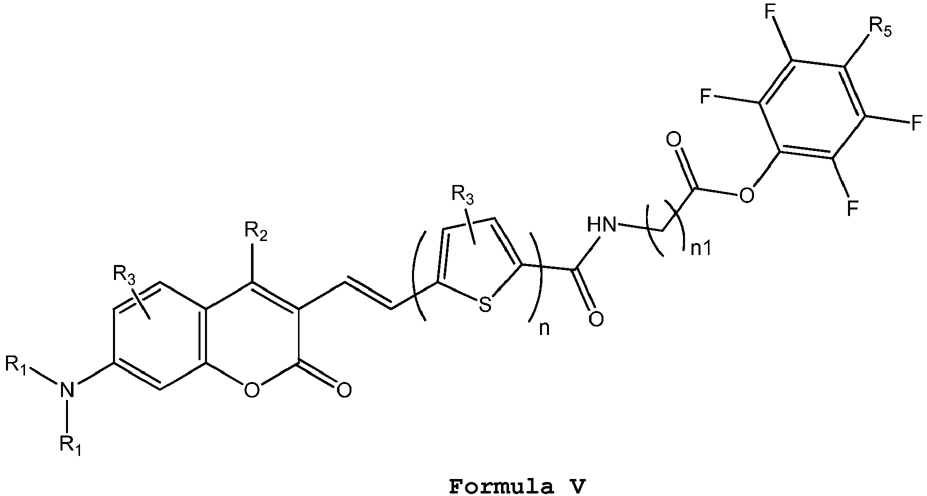 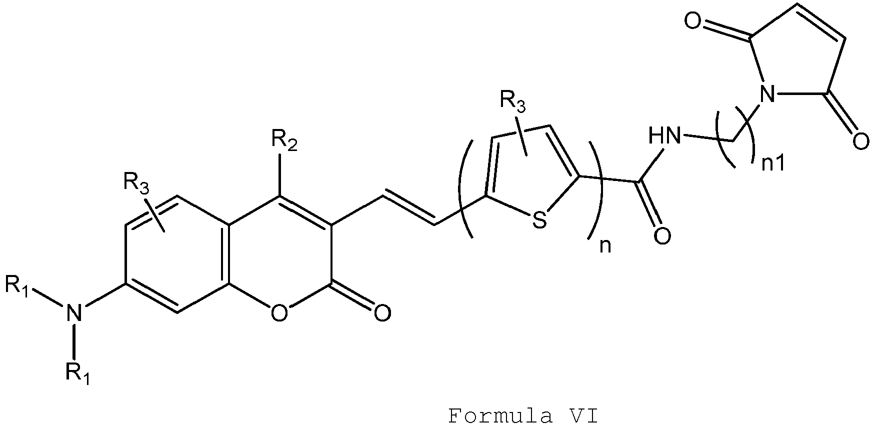 Wherein:
n is 1 or 2;
n1 is 1, 2, 3, 4 or 5;
R₁ is -(CH₂)ₙ-CH₃, and n is 0, 1, 2 or 3;
R₂ is -(CH₂)ₙ-CH₃, and n is 0 or 1;
R₃ is independently H or SO₃⁻ Na⁺; and
R₅ is H, F or SO₃⁻ Na⁺. 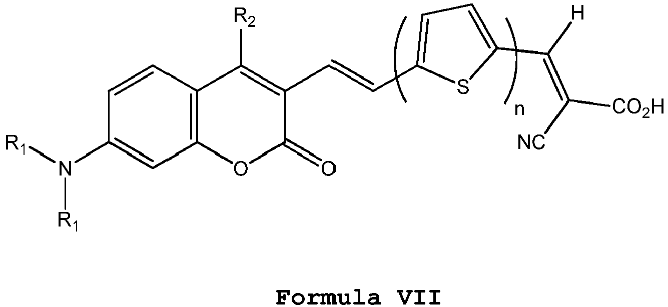 Wherein:
n is 1 or 2;
R₁ is -(CH₂)ₙ-CH₃, and n is 0, 1, 2 or 3;
R₂ is -(CH₂)ₙ-CH₃, and n is 0 or 1.

**3.** Fluorescent coumarins according to claim 1 or 2 having the general formula (II), (III), (IV), (V) and (VI) for producing dyes for tagging biomolecules.

**4.** Fluorescent coumarins according to claim 1 or 2 having the general formula (VII) producing dye-sensitized solar cells (DSSCs).

**5.** Compounds of formulae (VIII), (IX), (X), (XI) and (XII) as intermediary compounds for producing fluorescent vinyl tiophene and/or bitiophene coumarins of general formula I. 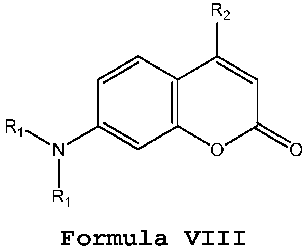 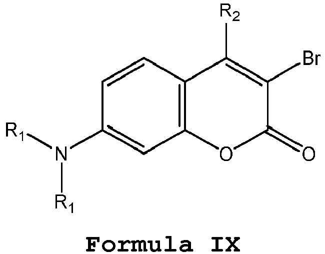 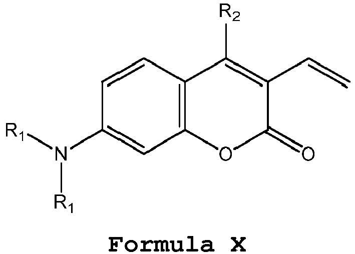 wherein:
R₁ is -(CH2)n-CH3, wherein n is 0, 1, 2 or 3; and
R₂ is -(CH2)n-CH3, wherein n is 0 or 1, 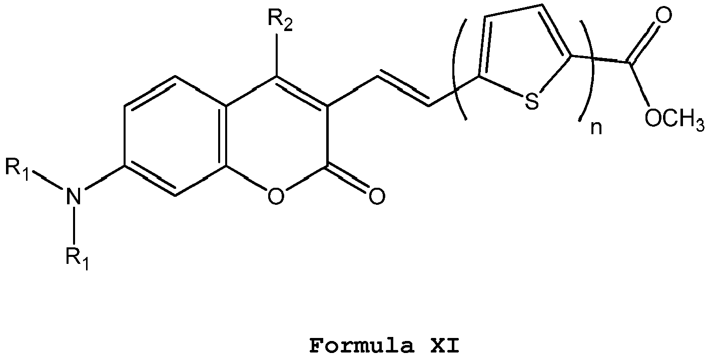
Wherein:
n is 1 or 2;
R₁ is -(CH2)n-CH3, wherein n is 0, 1, 2 or 3; and
R₂ is -(CH₂)ₙ-CH₃, and n is 0 or 1. 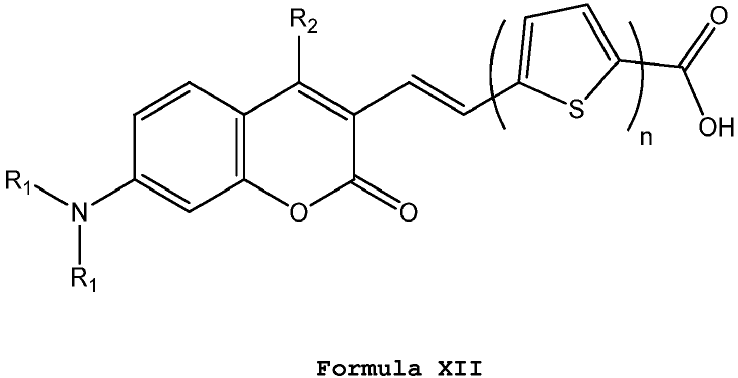
Wherein
n is 1 or 2;
R₁ is -(CH2)n-CH3, wherein n is 0, 1, 2 or 3; and
R₂ is -(CH₂)ₙ-CH₃, and n is 0 or 1. 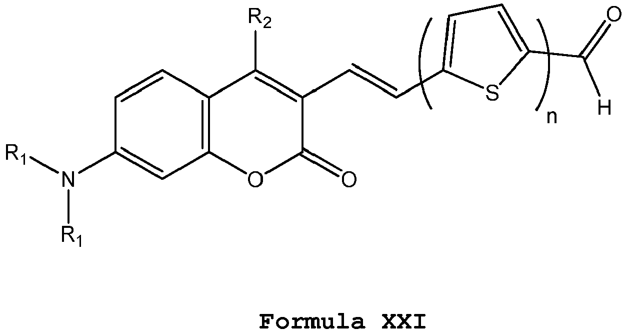
Wherein:
N is 1 or 2;
R₁ is -(CH₂)ₙ-CH₃, and n is 0, 1, 2 or 3;
R₂ is -(CH₂)ₙ-CH₃, and n is 0 or 1.

**5.** A **biosensor** for detecting, identifying and/or quantifying biomolecules comprising at least one of the compounds of formulae (II), (III), (IV), (V) and (VI) as described in any of the claims 1 to 3.

**6.** A **DSSC** for generating electrical energy comprising at least one of the compounds of formulae (VII), as described in any of the claims 1,2 or 4.

**7.** A **method** for preparing the **compounds of formula (I)** as described in claim 3 comprising the following steps:
a) **reacting a compound of formula (VIII)** 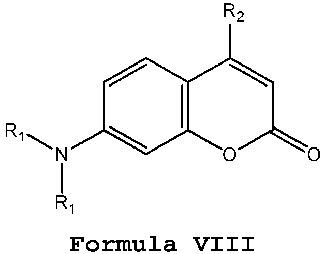 wherein:
R₁ is -(CH2)n-CH3, wherein n is 0, 1, 2 or 3; and
R₂ is -(CH2)n-CH3, wherein n is 0 or 1.
with bromine in a reaction solvent selected from the group comprising chloroform, dichloromethane, 1,2-dichloroethane, acetonitrile, a mixed solvent thereof and the like, under stirring at a temperature between 0°C and 5°C for 10 hours to 24 hours;
b) adding to the **reactional mixture** a base selected from the group comprising triethylamine, 4-(dimethylamino)pyridine, piperidine, piperazine, pyridine, 1,4-diazabicyclo[2.2.2]octane (DABCO), 1,8-diazabicyclo[5.4.0]undec-7-ene (DBU) and the like, and stirring the reactional mixture at a temperature between 20°C and 25°C for 30 minutes to 3 hours; and
evaporating the reaction mixture to dryness and purifying the residue **to produce a compound of formula (IX);** 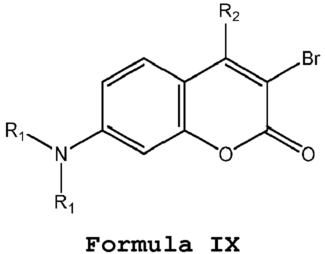 wherein:
R₁ is -(CH2)n-CH3, wherein n is 0, 1, 2 or 3; and
R₂ is -(CH₂)ₙ-CH₃, and n is 0 or 1.
b) **reacting compounds of formula (IX)** with potassium vinyltrifluoroborate and [1,1'-bis(diphenylphosphino)ferrocene]dichloropalladium(II) complex with dichloromethane in a reaction solvent selected from the group comprising n-propanol, 2-propanol, ethanol, tert-butanol, 2-butanol, acetonitrile, a mixed solvent thereof and the like, and stirring under inert atmosphere at a temperature between 78°C and 98°C for 15 minutes to 2 hours; and
evaporating the reaction mixture to dryness and purifying the residue **to produce a compound of formula (X)** 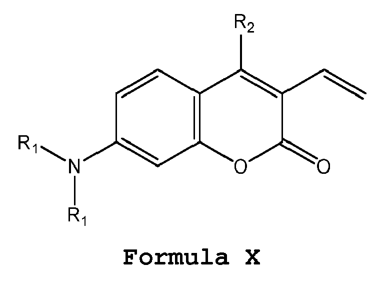 wherein:
R₁ is -(CH2)n-CH3, wherein n is 0, 1, 2 or 3; and
R₂ is -(CH₂)ₙ-CH₃, and n is 0 or 1.
d) **reacting compounds of formula (X)** with methyl 5-bromothiophene-2-carboxylate (or methyl 5'-bromo-[2,2'-bithiophene]-5-carboxylate),tetrakis(triphenylphosphine)palladium(0) and silver acetate in a reaction solvent selected from the group comprising N,N-dimethylformamide, dimethyl sulfoxide, a mixed solvent thereof and the like, and stirring under inert atmosphere at a temperature between 78°C and 98°C for 24 hours to 72 hours; and
evaporating the reaction mixture to dryness and purifying the residue to **produce compounds of formula (XI)** 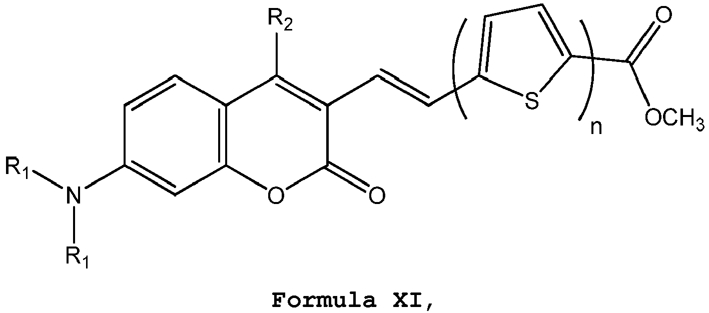 Wherein:
n is 1 or 2
R₁ is -(CH2)n-CH3, wherein n is 0, 1, 2 or 3; and
R₂ is -(CH₂)ₙ-CH₃, and n is 0 or 1.
e) **reacting a compounds of formula (XI)** with 1 M sodium hydroxide in a reaction solvent selected from the group comprising 1,4-dioxane, N,N-dimethylformamide, dimethyl sulfoxide, a mixed solvent thereof and the like, and stirring at a temperature between 50°C and 70°C for 1 hour to 2 hours and neutralizing with HCl (10%) solution; and evaporating the reaction mixture to dryness and purifying the residue to **produce compounds of formula (XII)** 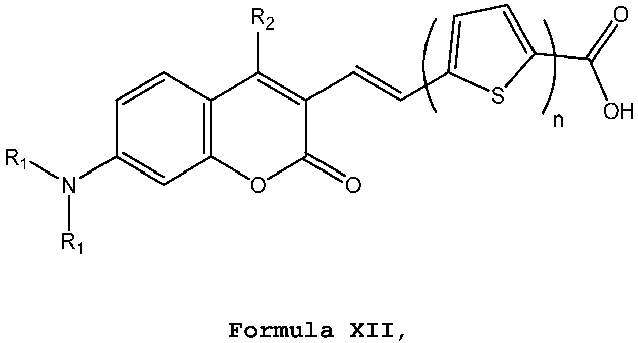 wherein
n is 1 or 2;
R₁ is -(CH2)n-CH3, wherein n is 0, 1, 2 or 3; and
R₂ is -(CH₂)ₙ-CH₃, and n is 0 or 1.
f) **reacting compounds of formula (XII)** with *N-*hydroxysuccinimide, and N,N'-dicyclohexylcarbodiimide in a reaction solvent selected from the group comprising acetonitrile and *N,N*-dimethylformamide, or dimethyl sulfoxide, a mixed solvent thereof and the like, and stirring at a temperature between 20°C and 26°C for 24 hours to 72 hours; and
evaporating the reaction mixture to dryness and purifying the residue to **produce compounds of formula (XIII).** 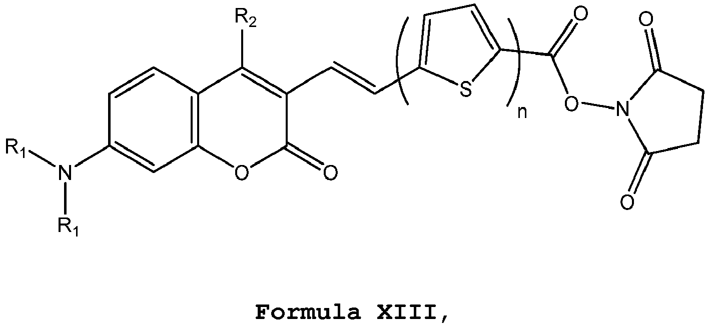 wherein:
n is 1 or 2;
R₁ is -(CH2)n-CH3, wherein n is 0, 1, 2 or 3; and
R₂ is -(CH₂)ₙ-CH₃, and n is 0 or 1.
g) **reacting compounds of formula (XIII)** with fuming sulfuric acid (30% SO3), and stirring at a temperature between 20 °C and 26 °C for 30 minutes to 1 hour; and adding NaCl until no further precipitate formation; and recrystallizing the reaction mixture in acetonitrile to **produce compounds of formula (II).** 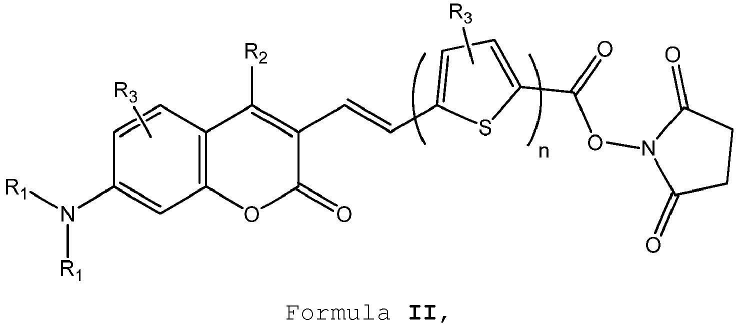 wherein:
n is 1 or 2
R₁ is -(CH2)n-CH3, wherein n is 0, 1, 2 or 3; and
R₂ is -(CH₂)ₙ-CH₃, and n is 0 or 1.
R₃ is independently H or SO₃⁻ Na⁺
h) **reacting compounds of formula (XII)** with 4-sulfotetrafluorophenol sodium salt (or pentafluorophenol, or 2,3,5,6-tetrafluorophenol), and N,N'-dicyclohexylcarbodiimide in a reaction solvent selected from the group comprising acetonitrile and N,N-dimethylformamide, or dimethyl sulfoxide, a mixed solvent thereof and the like, and stirring at a temperature between 20°C and 26°C for 24 hours to 72 hours; and
evaporating the reaction mixture to dryness and purifying the residue to **produce compounds of formula (XIV).** 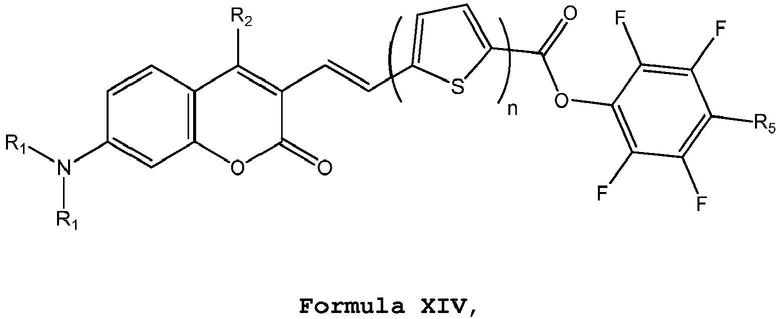 wherein:
n is 1 or 2
R₁ is -(CH2)n-CH3, wherein n is 0, 1, 2 or 3; and
R₂ is -(CH₂)ₙ-CH₃, and n is 0 or 1.
R₅ are H, F or SO₃⁻ Na⁺.
i) **reacting compounds of formula (XIV)** with fuming sulfuric acid (30% SO3), and stirring at a temperature between 20 °C and 26 °C for 30 minutes to 1 hour; and adding NaCl until no further precipitate formation; and recrystallizing the reaction mixture in acetonitrile to **produce compounds of formula (III).** 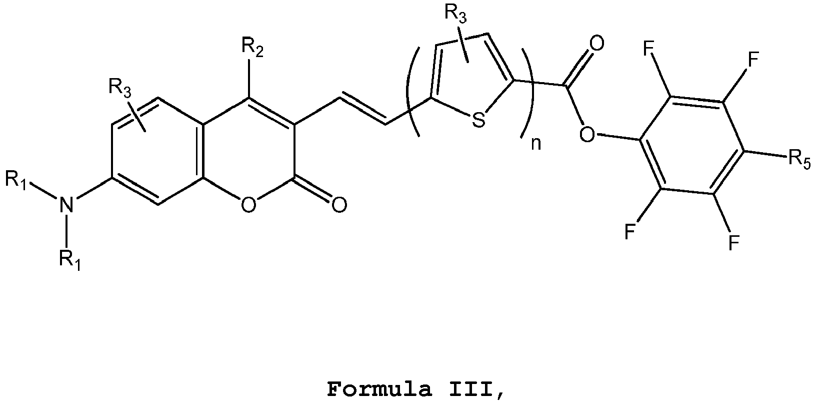 wherein:
n is 1 or 2;
R₁ is -(CH2)n-CH3, wherein n is 0, 1, 2 or 3;
R₂ is -(CH₂)ₙ-CH₃, and n is 0 or 1;
R₃ is independently H or SO₃⁻ Na⁺; and
R₅ is H, F or SO₃⁻ Na⁺.
j) **reacting compounds of formula (XII)** with methyl 2-aminoacetate (or methyl 3-aminopropanoate, or methyl 4-aminobutanoate, or methyl 5-aminopentanoate, or methyl 6-aminohexanoate), and N,N'-dicyclohexylcarbodiimide in a reaction solvent selected from the group comprising acetonitrile and N,N-dimethylformamide, or dimethyl sulfoxide, a mixed solvent thereof and the like, and stirring at a temperature between 20 °C and 26 °C for 24 hours to 72 hours; and
evaporating the reaction mixture to dryness and purifying the residue to **produce compounds of formula (XV).** 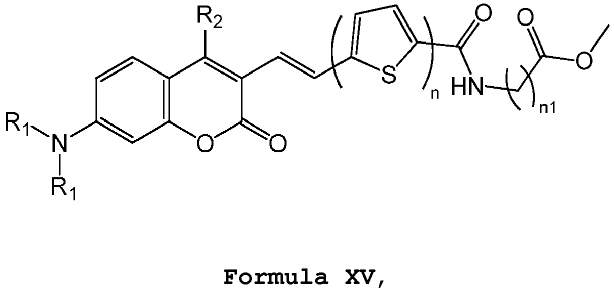 Wherein:
n is 1 or 2;
n₁ is, 1, 2, 3, 4 or 5;
R₁ is -(CH2)n-CH3, wherein n is 0, 1, 2 or 3; and
R₂ is -(CH₂)ₙ-CH₃, and n is 0 or 1.
l) **reacting compounds of formula (XV)** with 1 M sodium hydroxide in a reaction solvent selected from the group comprising 1,4-dioxane, N,N-dimethylformamide, dimethyl sulfoxide, a mixed solvent thereof and the like, and stirring at a temperature between 50 °C and 70 °C for 1 hour to 2 hours and neutralizing with HCl (10%) solution; and
the reaction mixture to dryness and purifying the residue to **produce compounds of formula (XVI).** 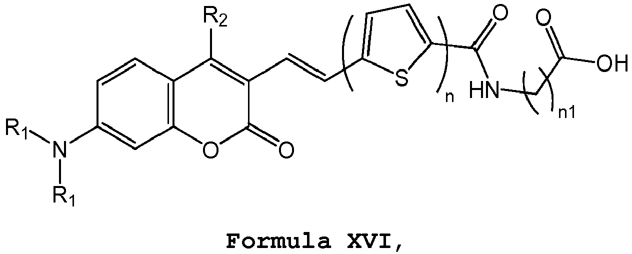 wherein:
n is 1 or 2;
n₁ is, 1, 2, 3, 4 or 5;
R₁ is -(CH2)n-CH3, wherein n is 0, 1, 2 or 3; and
R₂ is -(CH₂)ₙ-CH₃, and n is 0 or 1.
m) **reacting compounds of formula (XVI)** with *N-*hydroxysuccinimide, and N,N'-dicyclohexylcarbodiimide in a reaction solvent selected from the group comprising acetonitrile and *N,N*-dimethylformamide, or dimethyl sulfoxide, a mixed solvent thereof and the like, and stirring at a temperature between 20 °C and 26 °C for 24 hours to 72 hours; and
evaporating the reaction mixture to dryness and purifying the residue to **produce compounds of formula (XVII).** 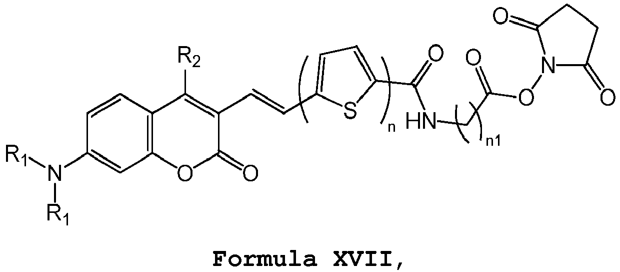 wherein:
n is 1 or 2;
n₁ is, 1, 2, 3, 4 or 5;
R₁ is -(CH2)n-CH3, wherein n is 0, 1, 2 or 3; and
R₂ is -(CH₂)ₙ-CH₃, and n is 0 or 1.
n) **reacting compounds of formula (XVII)** with fuming sulfuric acid (30% SO3), and stirring at a temperature between 20 °C and 26 °C for 30 minutes to 1 hour; and adding NaCl until no further precipitate formation; and recrystallizing the reaction mixture in acetonitrile to **produce compounds of formula (IV)**. 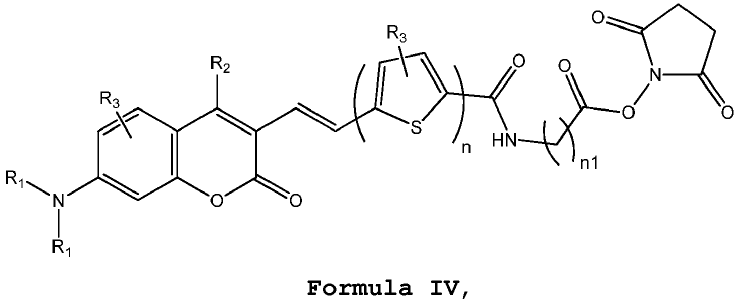 wherein:
n is 1 or 2;
n₁ is, 1, 2, 3, 4 or 5;
R₁ is -(CH2)n-CH3, wherein n is 0, 1, 2 or 3;
R₂ is -(CH₂)ₙ-CH₃, and n is 0 or 1; and
R₃ is independently H or SO₃⁻ Na⁺.
o) **reacting compounds of formula (XVI)** with 4-sulfotetrafluorophenol sodium salt (or pentafluorophenol, or 2,3,5,6-tetrafluorophenol), and N,N'-dicyclohexylcarbodiimide in a reaction solvent selected from the group comprising acetonitrile and N,N-dimethylformamide, or dimethyl sulfoxide, a mixed solvent thereof and the like, and stirring at a temperature between 20 °C and 26 °C for 24 hours to 72 hours; and evaporating the reaction mixture to dryness and purifying the residue to **produce compounds of formula (V).** 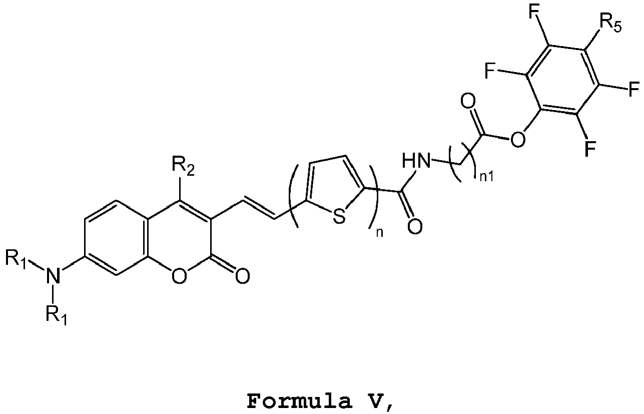 wherein:
n is 1 or 2;
n₁ is, 1, 2, 3, 4 or 5;
R₁ is -(CH2)n-CH3, wherein n is 0, 1, 2 or 3; and
R₂ is -(CH₂)ₙ-CH₃, and n is 0 or 1.
p) **reacting compounds of formula** (XII) with methanediamine (or ethane-1,2-diamine, or propane-1,3-diamine, or butane-1,4-diamine, or pentane-1,5-diamine), and N,N'-dicyclohexylcarbodiimide in a reaction solvent selected from the group comprising acetonitrile and N,N-dimethylformamide, or dimethyl sulfoxide, a mixed solvent thereof and the like, and stirring at a temperature between 20 °C and 26 °C for 24 hours to 72 hours; and evaporating the reaction mixture to dryness and purifying the residue to **produce compounds of formula (XVIII).** 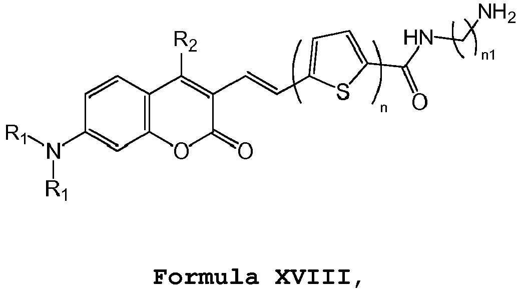 wherein:
n is 1 or 2;
n₁ is, 1, 2, 3, 4 or 5;
R₁ is -(CH2)n-CH3, wherein n is 0, 1, 2 or 3; and
R₂ is -(CH₂)ₙ-CH₃, and n is 0 or 1.
q) **reacting compounds of formula (XVIII)** with maleic anhydride, in a reaction solvent selected from the group comprising acetonitrile, or N,N-dimethylformamide, or dimethyl sulfoxide, a mixed solvent thereof and the like, and stirring at a temperature between 20 °C and 26 °C for 3 hours to 24 hours; and
evaporating the reaction mixture to dryness and purifying the residue to **produce compounds of formula (XIX).** 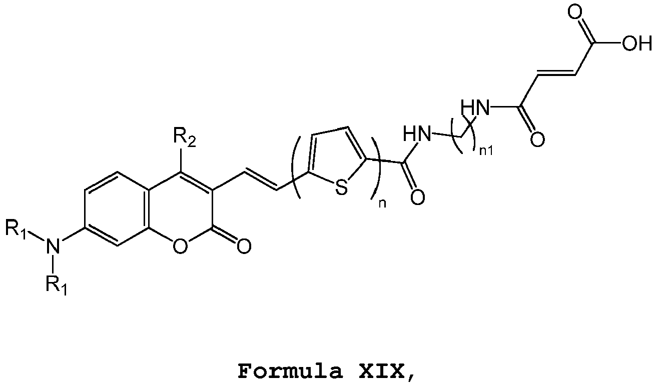 wherein:
n is 1 or 2;
n₁ is, 1, 2, 3, 4 or 5;
R₁ is -(CH2)n-CH3, wherein n is 0, 1, 2 or 3; and
R₂ is - (CH₂)ₙ-CH₃, and n is 0 or 1.
r) **reacting compounds of formula (XIX)** with acetic anhydride and triethylamine, in a reaction solvent selected from the group comprising acetonitrile, or N,N-dimethylformamide, or dimethyl sulfoxide, a mixed solvent thereof and the like, and stirring at a temperature between 60 °C and 100 °C for 24 hours to 72 hours; and
evaporating the reaction mixture to dryness and purifying the residue to **produce compounds of formula (XX).** 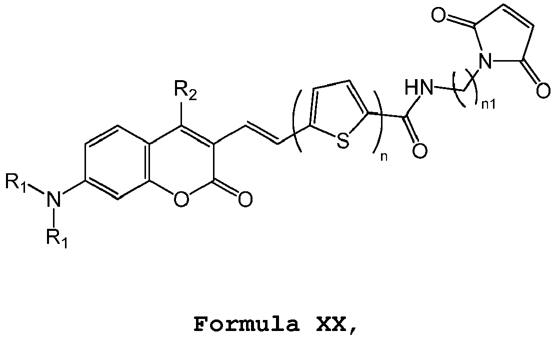 wherein:
n is 1 or 2;
n₁ is, 1, 2, 3, 4 or 5;
R₁ is -(CH2)n-CH3, wherein n is 0, 1, 2 or 3; and
R₂ is - (CH₂)ₙ-CH₃, and n is 0 or 1.
s) **reacting compounds of formula (XX)** with fuming sulfuric acid (30% SO3), and stirring at a temperature between 20 °C and 26 °C for 30 minutes to 1 hour; and adding NaCl until no further precipitate formation; and recrystallizing the reaction mixture in acetonitrile to **produce compounds of formula (VI).** 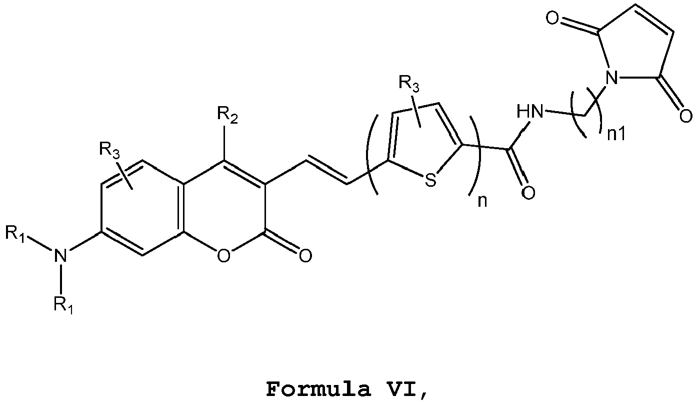 Wherein:
n is 1 or 2;
n₁ is, 1, 2, 3, 4 or 5;
R₁ is -(CH2)n-CH3, wherein n is 0, 1, 2 or 3;
R₂ is -(CH₂)ₙ-CH₃, and n is 0 or 1; and
R₃ is independently H or SO₃⁻ Na⁺.

**8.** A **method** for preparing the **compounds of formulae (I)** as described in claim 4 comprising the following steps:
a) **reacting a compound of formula (X)** with 5-bromothiophene-2-carbaldehyde (or 5'-bromo-[2,2'-bithiophene]-5-carbaldehyde), tetrakis(triphenylphosphine)palladium(0) and silver acetate in a reaction solvent selected from the group comprising N,N-dimethylformamide, dimethyl sulfoxide, a mixed solvent thereof and the like, and stirring under inert atmosphere at a temperature between 78 °C and 98 °C for 24 hours to 72 hours; and evaporating the reaction mixture to dryness and purifying the residue to **produce compounds of formula (XXI).** 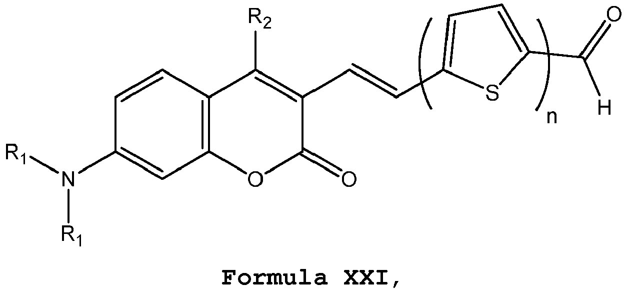 Wherein:
n=1 or 2;
R₁ is -(CH₂)ₙ-CH₃, and n is 0, 1, 2 or 3; and
R₂ is -(CH₂)ₙ-CH₃, and n is 0 or 1.
b) **reacting compounds of formula (XXI)** with cyanoacetic acid and piperidine in a reaction solvent selected from the group comprising acetonitrile or N,N-dimethylformamide, or dimethyl sulfoxide, a mixed solvent thereof and the like, and stirring at a temperature between 70 °C and 100 °C for 1 hour to 6 hours; and
evaporating the reaction mixture to dryness and purifying the residue to **produce compounds of formula (VII).** 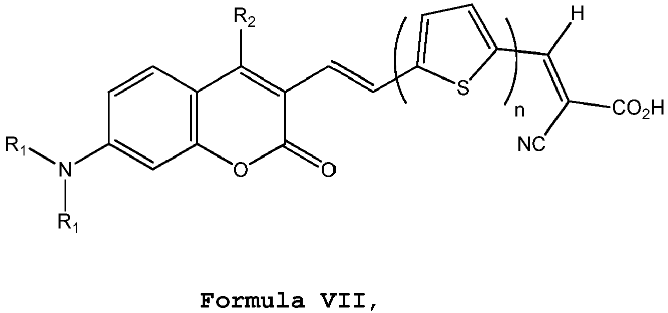 Wherein:
n=1 or 2;
R₁ is -(CH₂)ₙ-CH₃, and n is 0, 1, 2 or 3; and
R₂ is -(CH₂)ₙ-CH₃, and n is 0 or 1.

**9.** The use of fluorescent compounds 7-alkylamino-3-vinyl tiophene and/or 7-alkylamino-3-vinyl ditiophene coumarin of formulae **(II), (III), (IV), (V) and (VI)** as described claim 3 as dyes for tagging biomolecules.

**10.** The use of fluorescent compounds 7-alkylamino-3-vinyl tiophene and/or 7-alkylamino-3-vinyl ditiophene coumarin of formula (VII) as photosensitizers for producing dye-sensitized solar cells.
